# EUROPEAN PATENT SPECIFICATION

(11) **EP 2 445 615 B1**
(45) Date of publication and mention of the grant of the patent: **17.05.2017**
(21) Application number: 10724641.5
(22) Date of filing: 07.06.2010
(51) Int. Cl.: B01D 63/08

(54) **MICROFLUIDIC DEVICES FOR DIALYSIS**
MIKROFLUIDISCHE VORRICHTUNGEN FÜR DIALYSE
DISPOSITIFS MICROFLUIDIQUES POUR DIALYSE

(30) Priority: 24.06.2009 US 220117 P
(43) Date of publication of application: 02.05.2012
(73) Proprietor: Oregon State University, Corvallis, OR 97331-2140 (US); Outset Medical, Inc., Portland OR 97227 (US)
(72) Inventor: DROST, Kevin, Corvallis OR 97331-6001 (US); JOVANOVIC, Goran, Nadezda, Corvallis OR 97330 (US); MILLER, Richard, T., Corvallis, OR 97330 (US); ANDERSON, Eric, K., Corvallis OR 97330 (US); WARNER-TUHY, Alana, Corvallis OR 97330-6564 (US); CURTIS, James, R., Portland OR 97214 (US); JOHNSON, Bruce W., Corvallis OR 97330 (US); WRAZEL Julie S., Corvallis OR 97330 (US)
(74) Representative: Vossius & Partner Patentanwälte Rechtsanwälte mbB
(86) International application number: PCT/US2010/037621
(87) International publication number: WO 2010/151419

(56) References cited:
- EP-A2- 0 324 922
- WO-A1-02/076529
- WO-A1-2006/042079
- GB-A- 1 289 738
- US-A- 4 080 295
- US-A- 4 115 273
- US-A- 4 310 416
- US-A- 5 094 749
- US-A1- 2005 202 557
- US-A1- 2009 211 977

## Description

### FIELD

This disclosure concerns microfluidic devices and methods for their use and manufacture.

### BACKGROUND

The advent of microfluidics technology holds significant promise for heat and mass transfer applications. For instance, there are a number of important systems that require species separation, heat addition or removal, or a combination thereof. Examples include dialysis of blood, separation of potable water from contaminated water sources, pasteurization of water, and miniaturized heat exchange.
Hemodialysis, the purification of blood external to the body, is a process used to treat renal failure. The chemical composition of blood must be controlled to perform its essential functions of bringing nutrients and oxygen to the cells of the body, and carrying waste materials away from those cells. Blood contains particles of many different sizes and types, including cells, proteins, dissolved ions, and organic waste products. Some of these particles, including proteins such as hemoglobin and albumin, are essential for the body to function properly. Others, such as urea, a waste product from protein metabolism, and large molecule waste products that only hemodiafiltration can remove, must be removed from the blood. Otherwise, they accumulate and interfere with normal metabolic processes. Still other particles, including many of the simple ions dissolved in the blood, are required by the body in certain concentrations that must be tightly regulated, especially when the intake of these substances varies. Membrane technologies are typically used to dialyze blood with dialysate having carefully controlled concentrations of various salts. There is a need to improve the mechanisms associated with dialysis to more efficiently and more cost-effectively dialyze and diafiltrate blood. In addition, for dialysis outside of a clinic setting, such as at home during the daytime or at night, there is a need to dialyze blood at lower flow rates (significantly lower than the minimum blood flow rates for conventional dialysis systems) over longer treatment times, both of which assist in reducing the amount of blood that is outside the patient's body at any given time, thus reducing the risk of potential blood loss or damage and increasing patient safety.

WO 2006/042079 Alis directed to a dialyser with improved efficiency of mass transfer across a dialysis membrane using microchannel separation.

US 2005/0202557 A1 describes an apparatus which duplicates the functionality of a physiological system, the apparatus including first and second layers each defining upper and lower surfaces, wherein each layer can comprise a mold and a semi-permeable membrane can be secured to the first and second molds such that the upper surface of the membrane is secured adjacent to the lower surface of the first mold and the lower surface of the membrane is secured adjacent to the upper surface of the second mold.

US 4,310,416 A describes a plate type fluid treatment apparatus for filtration and separation of a fluid, transferring substances between fluids through membranes or passing specific substances contained in the fluid through membranes.

EP 0 324 922 A2 discloses an arrangement for the diffusion of substances between two fluids via semi-permeable membranes which are arranged in a stack separated by supporting plates having a first plane face and a second plane face and comprising through-openings connected to an inlet and outlet, respectively.

### SUMMARY

The present disclosure concerns microfluidic device as defined by independent claim 1. The device is configured for mass transfer by dialysis. For instance, certain disclosed embodiments incorporate semi-permeable membranes into microfluidic transfer devices to transfer target substances from one fluid to another, such as pursuant to a hemodialysis procedure. The microfluidic transfer device is sometimes described herein for use in a hemodialysis procedure although the microfluidic transfer device is suitable for other uses.

Mass transfer rates through membranes in conventional systems may be affected by diffusion rates through the relatively thick boundary layer between the bulk fluid and the membrane surface. Increasing fluid velocity near the membrane surface, for example by stirring, is a common method of decreasing the boundary layer thickness, thus the effective diffusion length. Membrane separation in a microfluidic device overcomes this difficulty using a microfluidic configuration such as microchannels or flow fields. The reduction in channel depth of a microfluidic device reduces may affect the mass transfer limitation caused by diffusion through the bulk fluid. However, manufacturing microfluidic devices often involves complicated and slow processes that make commercial production less feasible.

Another problem with membrane separation using microfluidic devices is the inherent limitations on the volume that can be processed. Previously disclosed microfluidic membrane devices overcome this difficulty by stacking multiple separators in parallel to reach desired volumetric flow rates. Such microfluidic membrane devices are disclosed in U.S. Application No.11/243,937; U.S. Publication No. 2008/0093298; and U.S. Publication No. 2008/0108122, which are incorporated herein by reference. Additionally, cross contamination is a significant risk in fluid separation processes. This application is directed to embodiments of microfluidic devices that mitigate the risk of cross contamination. For example, certain disclosed embodiments use compression sealing to reduce or substantially eliminate cross contamination of fluids. Integrated into certain disclosed embodiments is a full compression seal around each fluid containing region, thereby preventing cross contamination. Moreover, the device can be manufactured using conventional low cost, high volume techniques.

Microfluidics and heat transfer technology have been described in U.S. Patent No. 6,793,831; U.S. Patent No. 6,688,381; U.S. Patent No. 6,672,502; U.S. Patent No. 5,813,235; U.S. Publication No. 2008/0006040; and U.S. Publication No. 2004/0157096, which are incorporated herein by reference.

One embodiment of the disclosed device comprises plural laminae, at least one lamina having a front side defining front side features and a back side defining backside features. The front side features may be fluidly connected to the back side features by at least one via. The laminae are interleaved by at least one transfer layer. The features on the front side may be, for instance, an inlet header and an outlet header. The features on the backside may be one or more microchannels or a flow field. In one embodiment the inlet header may comprise an inlet port and the outlet header may comprise an outlet port.

To scale the device to the desired volumetric capacity, the device may comprise a stack of plural subunits capable of performing a unit operation, such as fluid purification. Each subunit has a first lamina, a second lamina, and a transfer layer positioned between the back side of the first lamina and the back side of the second lamina. The device may further comprise gaskets positioned between the subunits. Each lamina comprises a first side, a second side, and a flow field or at least one, and typically plural, microchannels. The plural subunits are aligned and stacked. The stack may be positioned between a first compression plate and a second compression plate. The stack may then be compressed to affect a substantial seal of all fluid containing regions.

Gasket designs for various embodiments may have a cutout for fluid to communicate between layers and/or a cutout over the fluid inlets and outlets to prevent the gasket material from sagging into the inlet and occluding flow. The transfer layers in the various embodiments may comprise cut-outs in the areas corresponding to the inlets headers and the outlets headers of adjacent layers. Similar to the gasket this prevents the transfer layer from collapsing into the headers and occluding flow.

Individual lamina may have features on the front side and the back side, the features fluidly connected by a via or vias. For instance, the laminae may comprise an inlet and an outlet on the front side and microchannels or one or more flow fields on the back side. The inlet and outlet may be associated with fluid headers or manifolds, and may be fluidly connected to opposite ends of the plural microchannels or the flow field by a first via or vias and a second via or vias positioned at opposite ends of the microchannels or flow field. The fluid inlets and outlets may define support columns for preventing adjacent laminae from sagging into the inlets and outlets and occluding flow.

Vias connecting the front side and the back side may be orthogonal to the microchannels or flow field. However, it may be desirable for the vias to intersect the laminae at a non-orthogonal angle. The non-orthogonal angle may be greater than zero and less than 90, but more typically is between about 45° and about 90°. Non-orthogonal vias can provide various benefits, such as by minimizing areas of fluid stagnation or gas entrapment.

The disclosed device may be configured various ways. For instance, in one embodiment of the disclosed device, the subunits are substantially identical with respective laminae in the stack in substantially similar orientations. For instance, the subunits may all be oriented with the front side of the first lamina facing the same compression plate. Microchannels or the one or more flow fields of adjacent layers within a subunit may be parallel or orthogonal to each other. Subunits with parallel microchannels may be orthogonal to adjacent subunits to maintain a compression seal around the headers.

Adjacent subunits also may be mirror images of each other and may be oriented in the stack such that the front sides of the first laminae of adjacent subunits face each other and the front sides of the second laminae of adjacent units face each other. One advantage of this embodiment is a simplified gasket design. Another advantage of this embodiment is reduced cross -contamination since layers having the same fluid are adjacent to each other.

Individual laminae may have more than one independent flow path. For example, a lamina may have a first flow path defined on a first side and a second flow path defined on an opposite side of the same lamina. In this embodiment the laminae are herein referred to as double-sided. For instance, in addition to the lamina described above, the back sides of the first lamina and the second lamina in the subunit may further comprise an inlet header and an outlet header, and the front sides of the first lamina and the second lamina may further comprise microchannels or one or more flow fields. The microchannels or one or more flow fields on the front side may be fluidly connected to the inlet header on the back side by one or more vias and fluidly connected to the outlet header on the backside by one or more vias. Rather than gaskets positioned between the subunits, transfer layers are positioned between the subunits. The transfer layers may be mass transfer layers.

This embodiment reduces the number of laminae required per transfer unit. The single sided embodiments required two laminae for each transfer unit, whereas the double-sided embodiment requires (n+1) laminae per transfer unit, where n is the number of desired transfer units. Thus for a device having one hundred transfer units, the double-sided embodiment only requires 101 laminae, whereas a single sided embodiment would require 200 laminae. To mitigate cross contamination between the fluid contained in the inlet and outlet on the front side and the fluid contained in the microchannels on the front side for this embodiment, the transfer layer may be substantially sealed to adjacent laminae between the inlet headers and microchannels or flow field and between the outlet headers and the microchannels or flow field. Likewise a risk of cross contamination exists on the back side, which may be similarly mitigated.

An embodiment employing through-cut microchannels or flow fields also ameliorates cross contamination risk while realizing the efficiency of a double-sided embodiment. In the through-cut embodiment, a substantial portion of the plural microchannels or flow fields of each lamina extend through the entire lamina thickness, namely from the front side to the back side to form through-cut microchannels or flow fields. In this way, the microchannels or flow fields simultaneously operate with two separation units.

The laminae of this embodiment comprise a front side and a back side, the front side having an inlet and an outlet. The inlet fluidly connects to the back side by one or more inlet vias. The one or more inlet vias fluidly connect to the through-cut microchannels or flow fields by partial thickness microchannels or flow fields on the backside side, and the outlet fluidly connects to the back side by one or more outlet vias. The outlet vias fluidly connect to the through-cut microchannels or flow fields by partial thickness microchannels on the back side. To add stability to the through-cut microchannels or flow fields, partial thickness orthogonal support structures may be spaced along the microchannels or flow fields. The through-cut laminae may be arranged in subunits having a first lamina, a second lamina, and a transfer layer. The device may have plural subunits and the microchannels of the first lamina may be substantially parallel or substantially orthogonal to the microchannels or flow fields of the second lamina, and the microchannels or flow fields of adjacent subunits may be orthogonal.

An embodiment of a microfluidic device may comprise plural laminae, at least one of the lamina having a front side defining an inlet header and an outlet header and a backside defining at least one backside feature. The inlet header and outlet header may be fluidly connected to at least one backside feature by one or more vias. One or more of the vias may be non-orthogonal. In this embodiment one or more transfer layers, defining cutouts corresponding to the inlet headers and outlet headers, interleave the laminae.

The present disclosure is also directed to a microfluidic device comprising plural laminae, at least one lamina having an inlet header and an outlet header, where transfer layers defining cutouts corresponding to the inlet header(s) and the outlet header(s) interleave the laminae. Like previous embodiments, this embodiment may be configured into subunits comprising a first lamina, a second lamina and a transfer layer. One or more subunits may be stacked between compression plates. The headers may define plural support posts and ports for fluid inlet and outlet. The laminae may define microchannels or one or more flow fields and the headers may also be shaped to provide approximately equal flow-path lengths between the inlet port and the outlet port. The headers may be, for example, approximately triangular.

This embodiment, as with previously disclosed embodiments may be configured as mirror image subunits, where the front sides of the first lamina of adjacent subunits face each other and the front sides of the second lamina of adjacent subunits face each other. Additionally, the laminae may be double sided laminae or may define through-cut microchannels as previously described. This embodiment may have various configurations, such as parallel or herringbone - pattern microchannels, or-one or more flow fields. Adjacent laminae may have microchannels or flow fields substantially parallel or substantially orthogonal to each other within a subunit or between subunits.

Another embodiment of the present disclosure is a microfluidic device comprising plural laminae having front sides and back sides, where at least one laminae defines a via for fluidly connecting the front side and the back side and where the via intersects the front side and the back side at a non-orthogonal angle. The device further comprises transfer layers interleaving the laminae. The laminae may have features on the front side and the back side, for instance inlet headers, outlet headers, and microchannels or a flow field, and may be arranged in one or more subunits. The subunits may be stacked between compression plates, and gaskets may be placed between subunits.

The plural microchannels of the various embodiments may be substantially parallel; however other microchannels patterns may be desirable depending on the application. A person of ordinary skill in the art will recognize that the microchannels may be a nested serpentine, fractally divergent, convergent, or of a herringbone pattern. Additionally, instead of microchannels, there can also be provided an array of support structures that could form a flow field. A flow field may have the added advantages of increasing the active surface area of the transfer layer, allowing fluid to flow around entrapped air bubbles or other occlusions, and improving fluid flow distribution.

Flow fields may be formed a pair of opposed, outer walls that define a flow space in which fluid flows. The support structures each comprise columns which may be prisms with circular bases, such as cylinders. However the bases of the prism may be also have a rectangle, tear-drop, triangle, ellipse, polygon, or tear-drop shape.

The flow field defines an array of support structures having gradient densities and varying sizes. One end of the flow field has larger and sparse support structures, gradually decreasing in size and increasing in density approaching the opposite end. Additionally, some or all of the flow field may be treated with a surface treatment to enhance flow dynamics. For instance the surface may be treated to render it hydrophilic to reduce air entrapment. Alternatively, it may be treated selectively to be hydrophobic in a support sparse area and hydrophilic in a support dense area, encouraging entrapped air to move through the sparse area to the dense area where wicking and mechanical forces can force the gas out. The support structures may also be randomly distributed throughout the flow field subject to design constraints.

Adjacent layers of the various embodiments may be oriented so that the microchannels or flow fields direct their respective fluids to flow in the same direction (concurrent), opposite directions (countercurrent), or at some angle relative to one another, such as orthogonally (crosscurrent). In the case of flow fields, the direction of flow may comprise the predominant direction of the fluid flowing through the flow field. Design considerations will balance the need for enhanced transfer driving force (temperature gradients or concentration gradients) with difficulties in construction and operation inherent in the configurations; such difficulties include alignment and pressure differentials between layers. A person of ordinary skill in the art will recognize that the preferred relative flow direction will depend on the application.

The disclosed embodiments are configured to perform mass transfer. The disclosed device is compatible with any membrane suitable for particular applications. Typical membranes for mass-transfer are semi-permeable membranes. Such membranes may be polymer, copolymer, metal, ceramic, composite, and/or liquid membranes. One example of a composite membrane is polysulfone-nanocrystalline cellulose composite membrane, which may be employed for a microchannel dialysis device, where metabolic waste is transferred from blood to dialysate. Gas-liquid contactor membranes may also be employed for transferring a substance between a liquid and gas. One such application would be oxygenation of blood, whereby the membrane allows transfer of carbon dioxide and oxygen, such that oxygen transfers to blood from oxygen or oxygen-enriched air, and carbon dioxide transfers from the blood to the gas. Fluid membranes may also be employed. Fluid membranes comprise a lamina having through cut microchannels containing fluid and a first and second membrane support positioned to contain fluid in the microchannels. The transfer layer may also be configured for a fuel cell where the transfer layer comprises a polymer electrolyte membrane positioned between a cathode layer and an anode layer.

The laminae used on the various embodiments may be any material which can be patterned with microchannel features or features capable of creating one or more flow fields. Typical laminae materials include polymers, metals, metal alloys, super alloys, or intermetallics. Suitable polymers include, without limitation, polycarbonate, polyethylene terephthalate (PET), polyether imide (PEI), Poly(methyl methacrylate) (PMMA), or a halogenated polyethylene such as poly(tetrafluoroethylene) (PTFE). Suitable metals or metal alloys include, without limitation, stainless steel, copper, titanium, nickel, or aluminum.

Microchannel/flow field depth generally may be as shallow as possible while still allowing the passage of fluid through the microchannel. The shallow depth should be consistent with, or calibrated with, the diffusion or conduction length, so the shallower the microchannel/flow field, the shorter the diffusion or conduction path, thus increasing transfer efficiency. The risk of flow occlusion may constrain the minimum depth due to depth irregularities, process fluid viscosities, process fluid contaminants, or biological concerns such as cell damage in the case of hemodialysis or blood oxygenation. To realize the benefit from the microchannel phenomenon, microchannel dimensions are typically greater than zero and less than about 1000 µm. More typically, the microchannels are greater than zero and less than about 400 µm, and even more typically greater than zero and less than about 100 µm, such as from about 10 µm to about 90 µm.

The embodiments of the present disclosure may be useful for transferring heat or mass from one fluid to another. By providing an embodiment of the present disclosure having a first lamina and a second lamina, the first and second lamina each having inlet headers and outlet headers connected to microchannels or one or more flow fields separated by a transfer layer, then providing a first fluid to the inlet header of the first lamina and a second fluid to the inlet header of the second lamina, the heat or mass will transfer from the first fluid to the second fluid or visa-versa, depending on the device configuration. For instance, by providing a device where the transfer layer is a semi-permeable membrane, such as polysulfone-nanocrystalline cellulose composite, and providing blood to one lamina and dialysate to the other lamina, substances in the blood will transfer to the dialysate.

Similarly, certain disclosed embodiments are useful for heat transfer systems. Incorporating thermally conductive layers into such devices allows heat to transfer from one fluid to another. Thus, in some embodiments, the transfer layers can be heat transfer layers.

The foregoing and other objects, features, and advantages of the invention will become more apparent from the following detailed description, which proceeds with reference to the accompanying figures.

### BRIEF DESCRIPTION OF THE DRAWINGS

FIG. 1 is a schematic view of a microfluidic transfer device having a through-flow via.
FIG. 2 is a perspective view of one embodiment of a single layer of the microfluidic transfer device.
FIG. 3 is a plan view of microfluidic flow field with wall segment supports.
FIG. 4 is a plan view of a microfluidic flow field with angled wall segments.
FIG. 5 is a schematic plan view of the juxtaposition of flow fields with angled wall segments.
FIG. 6A is a plan view of a microfluidic flow field with cylindrical supports.
FIG. 6B shows a top view of a pair of cylindrical supports.
FIG. 6C shows a side view of a pair of cylindrical supports.
FIG. 7 is a plan close-up view of a microfluidic flow field with tear-drop shaped support structures.
FIG. 8 is a plan close-up view of a microfluidic flow field having gradient support structure density and size.
FIG. 9 is a plan close-up view of a microfluidic flow field with randomly distributed support structures.
FIG. 10 is a partial perspective view of an assembled device showing fluid inlets and outlets.
FIG. 11 is a perspective view of two combined assembled devices with fluidic headers attached.
FIG. 12 is an assembly view of one embodiment of a micro fluidic transfer device with single-sided lamina.
FIG. 13 is a plan view of one embodiment of a lamina.
FIG. 14 is a perspective view of the assembled device shown in FIG. 6.
FIG. 15 is a detail view of the internal fluid flow paths in the device of FIG. 6.
FIG. 16 is a schematic plan view of the juxtaposition of fluid headers and microchannels of adjacent layers, having cross-current flow.
FIG. 17 is a partial schematic plan view of the juxtaposition of adjacent layers having the flow field shown in FIG. 3.
FIG. 18 is a detail view of the internal fluid flow paths of one embodiment having single-sided mirrored design.
FIG. 19 is a detail perspective view of the fluid flow paths of a one embodiment having a single-sided mirrored design with parallel microchannels.
FIG. 20 is a partial assembly view of one embodiment of a microfluidic transfer device having double sided laminae.
FIG. 21 is a plan view of a double-sided lamina.
FIG. 22 is a plan view of a transfer layer.
FIG. 23 is a detail view of the flow path of a microfluidic transfer device having double sided laminae.
FIG. 24 is a detail view of the flow path of a microfluidic transfer device having double sided laminae with concurrent flow.
FIG. 25 is a plan view of a lamina having through-cut microchannels.
FIG. 26 is a detail plan view of a lamina having through-cut microchannels with lateral supports.
FIG. 27 is a detail plan view of a lamina having through-cut microchannels with a herringbone pattern.
FIG. 28 is a detail perspective view of a lamina having through-cut microchannels with a herringbone pattern.
FIG. 29 is an assembly view of a microfluidic transfer device having through-cut laminae.
FIG. 30 is a detail view of the fluid flow path of a device having through-cut laminae.
FIG. 31 is a perspective view of a device having alternating parallel and orthogonal through-cut microchannels.
FIG. 32 is a plan view of the juxtaposition of the layers of a subunit incorporating a fluid membrane.
FIG. 33 is a schematic view of a device having fluid membranes.
FIG. 34 is a schematic view of a device having fuel cells.
FIG. 35 is a plan view of an embodiment of a lamina of a flow field dialyzer without header regions.
FIG. 36 is a plan view of another embodiment of a lamina of a flow field dialyzer without header regions.
FIG. 37 is a plan, schematic view of a pathway of lasers for forming a flow field.
FIG. 38 is a an enlarged view of a portion of a lamina where laser-formed channels intersect.
FIG. 39 is an enlarged view of a lamina surface showing undulating channels and pins formed between the channels.
FIG. 40 is an embodiment where alternating headerless laminae are stacked in a cross-current manner.

### DETAILED DESCRIPTION

### A. Definitions

Dialysate refers to an aqueous fluid having the proper solutes in the proper concentrations for blood dialysis.

Microchannel refers to a substantially bound space in a microfluidic device that allows the flow of fluid, the space having at least one dimension less than about 1000 µm.

Flow field means a micro fluidic pathway with an aspect ratio of about 10 or more, where the aspect ratio is defined as ratio of the width of the micro fluidic pathway and the depth of the micro fluidic pathway and fluid flows substantially in the direction of the length of the micro fluidic pathway.

Through-cut microchannel refers to a microchannel with a depth equal to the thickness of the lamina in which it is formed.

Single-sided laminae refers to laminae that have features, such as microchannel or flow field features, in which heat or mass transfer across the transfer layers occurs on one side only.

Double-sided laminae refers to laminae that have features, such as microchannel features or flow field features, on both sides.

Semi-permeable membrane refers to a membrane that allows passage of some substances through it while restricting others.

Via refers to a microchannel that allows fluid to flow through a lamina to fluidly connect microchannels or flow fields on the lamina front side with those on the lamina back side.

### B. Microfluidic Transfer Device Description

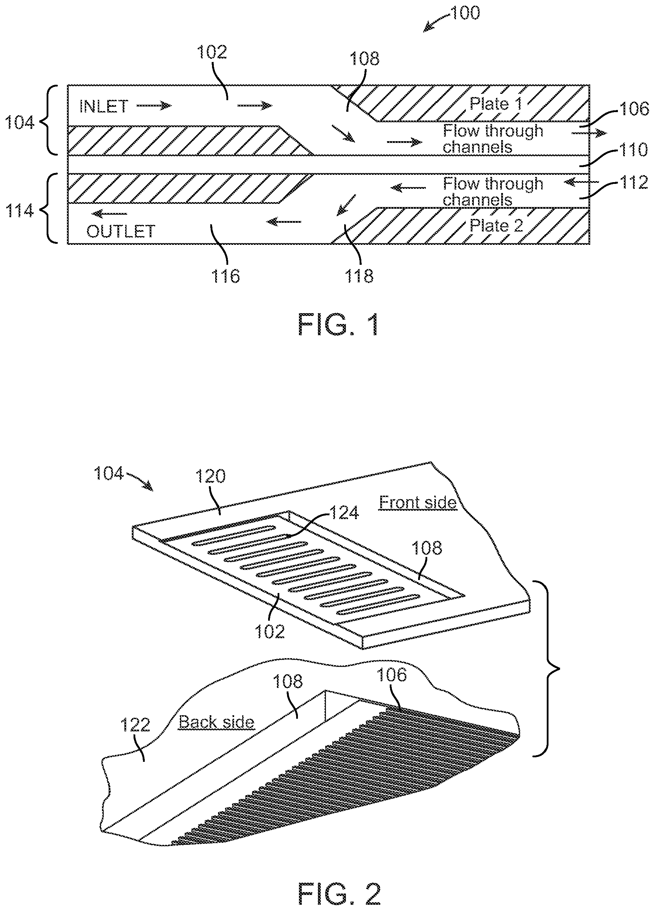

FIG. 1 depicts a counter current flow diagram 100 for a first fluid and a second fluid. Although the flow pathway is described in the context of a microchannel, a flow pathway may also be used through a flow field. Moreover, although various embodiments described herein are shown with microchannel configurations, each embodiment could be constructed and operate using a fluid flow field rather than a microchannel.

A first fluid enters microchannel inlet 102 and flows through upper lamina 104 to microchannel 106 by way of via 108 where the fluid contacts transfer layer 110. Concurrently, a second fluid in microchannel 112 contacts transfer layer 110 before flowing through lower lamina 114 to outlet 116 by way of via 118. Transfer layer 110 may be a semipermeable membrane chosen for the specific application to allow transfer of one or more substances from the fluid in microchannel 106 to the fluid in microchannel 112, or visa-versa. For example, the specific application may be a hemodialysis procedure.

The width of microchannels 106 and 112 will be the widest possible considering operating parameters and construction requirements, such as to substantially prevent the transfer layer 110 from sagging into the microchannels. The actual width will vary depending on certain factors, such as the rigidity of the transfer layer 110 and the pressure differential across the transfer layer. Typical microchannel widths are between 100 µm and 500 µm, and more typically between about 200 µm and about 400 µm.

For mass transfer devices, transfer layer 110 may be any material which allows selective transfer of a target substance(s) through the transfer layer. A person of ordinary skill in the art will recognize that the membrane selection will depend on other design criteria including, without limitation, the substance being transferred, other substances present in the fluids, the desired rate of transfer, the fluids carrying the substance, the fluid receiving the substance, operating temperature, and operating pressure. Suitable membranes may include, without limitation, polymer, copolymer, metal, ceramic, composites, polysulfone-nanocrystalline cellulose composite, gas-liquid contactor membranes, hollow fiber membranes, and fluid membranes.

Where the mass transfer device is a hemodialysis device, some suitable membranes for the transfer layer include without limitation, polysulphone, polyethersulfone, polyacrylanitrile, cellulose acetate, cellulose di-acetate, and cellulose tri-acetate.

Laminae 104 and 114 may be any material capable of being patterned with features useful for a particular application, such as vias and microchannels or such as support structures for flow fields. Lamina thickness may be between about 200 µm and about 1000 µm, with typical thicknesses being between about 300 µm and about 500 µm. Suitable lamina materials include, without limitation, polymers and metals. Examples of suitable polymeric materials include polycarbonate, polyethylene terephthalate (PET), polyether imide (PEI), poly(methyl methacrylate) (PMMA), and halogenated polyethylene such as poly(tetrafluoroethylene) (PTFE). Metal laminae may be any that can have desired features formed therein, such as materials that can be photo-chemically etched or otherwise machined to have desired features, including blind features. Examples include stainless steels, copper, titanium, nickel, and aluminum.

FIG. 2 shows a perspective view of one embodiment of a lamina design. Although FIG. 2 is described in the context of a microchannel, a similar header configuration may be used for fluid flowing into a flow field. The header side 120 of lamina 104 comprises inlet 102 for receiving a fluid and directing the fluid to via 108 where the fluid flows through the plate to the microchannel side 122 (or flow field side) of the plate. The fluid then flows through the microchannels 106 or flow field, where the fluid contacts the transfer layer, not shown. The inlet 102 has support structures 124 for preventing collapse of adjacent lamina into the inlet 102. FIG. 2 discloses microchannels 106 as plural parallel microchannels, however the present disclosure is not limited to this configuration.

### 1. Flow Fields

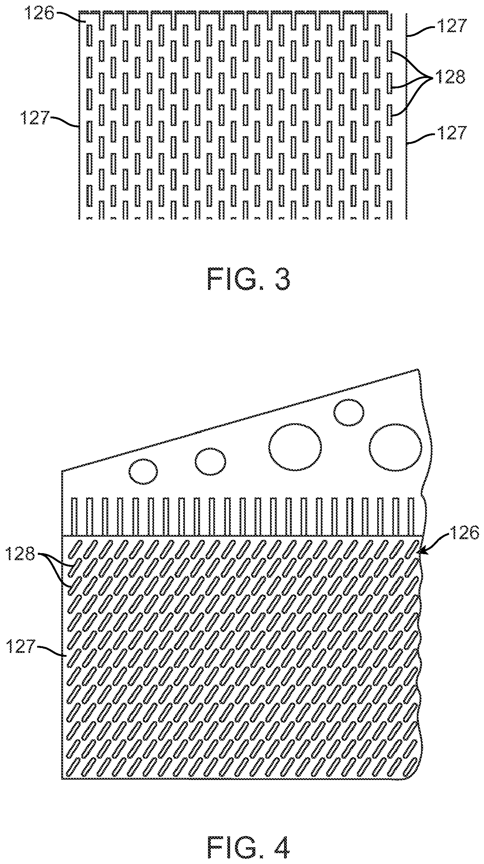

As mentioned, any of the embodiments may incorporate one or more flow fields rather than microchannels. FIG. 3 shows an embodiment utilizing a flow field 126 rather than the parallel microchannels used in the embodiment of FIGS. 1 and 2. The flow field 126 may be generally formed by a pair of opposed walls 127 that define the outer periphery of the flow field 126. A space is positioned between the walls and fluid flows within the space from an inlet toward an outlet. One or more discrete support structures, such as wall segments 128, are positioned in the space between the walls. The support structures at least partially function to provide support to adjacent lamina to prevent the lamina from collapsing on another. The support structures also prevent the membrane from collapsing into the flow path and blocking the flow of either blood or dialysate. The support structures may be arranged in a variety of spatial arrangements relative to one another. The support structures may have a variety of shapes and sizes and may be in the form of pins, wall segments, bumps, protrusions, etc.

The support structures differ from the elongated walls or dividers that form the microchannels in that the support structures do not define discrete, elongated flow pathways. Rather, a plurality of the support structures are positioned in the general flow space between the opposed walls 127 without specifically guiding the fluid in a particular direction. The support structures permit more freedom of flow direction for the fluid relative to the finely-guided directional flow of the microchannels.

In addition, the discrete, spaced-apart nature of the support structures results in exposure of more transfer layer surface than where contiguous microchannel dividers are used. Exposure of more of the interposed transfer layer to the fluids to be dialyzed, for example, improves overall device efficiency. A person of ordinary skill in the art will recognized that it is desirable to maximize the area of the transfer layer exposed to the fluid while maintaining the integrity of the transfer layer with sufficient support structures so that the transfer layer does not collapse into a portion of the flow field. Moreover, the flow field embodiments mitigate flow occlusion cause by entrapped air bubbles by allowing fluid to flow around the air bubbles, which may not occur as readily in the more constricted volume of a microchannel because the bubble may be of a size to significantly block the flow in a particular channel.

In the example of FIG. 3, the support structures are in the form of wall segments 128 comprised of rectangular- or prismic-shaped bodies that extend upwardly from the surface of the lamina. The wall segments 128 are positioned in groups such that a single group forms a column from the viewpoint of FIG. 3. The entire flow field 126 includes a series of columns. Each column has a plurality of wall segments 128 aligned end-to-end and spaced from one another within the column. Each of the columns is spaced from an adjacent column. The spacing between wall segments may vary within a single column, as can the spacing between one column and an adjacent column. Moreover, the wall segments 128 can be arranged in other spatial patterns and are not limited to being arranged in column patterns.

FIG. 4 shows another example of a flow field 126 with the wall segments 128 angled slightly relative to the long axes of the opposed walls 127. The magnitude of the angle of the wall segments relative to the opposed walls 127 may vary. All of the wall segments 128 may be oriented at the same angle. Alternately, the angle may vary from one wall segment 128 to another wall segment 128 such that one or more of the wall segments 128 may be oriented at one angle while other wall segments may be oriented at a different angle.

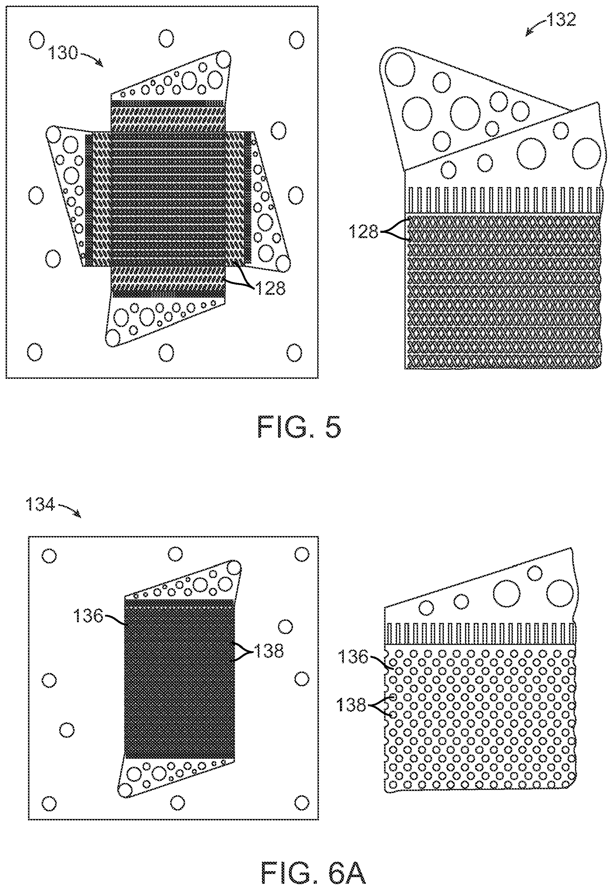

Angling the wall segments 128 results in an alignment tolerant design when the device is assembled for concurrent or countercurrent flow. It can be desirable for one wall segment to be positioned at least partially atop another wall segment when the adjacent layers are stacked in order to provide proper structural support between the layers in the stack. In this regard, the wall segments 128 can have relative sizes and shapes and can also be arranged in patterns to maximize the likelihood of the wall segments aligning atop one another when the layers are stacked. FIG. 5 shows a juxtaposition of adjacent layers in cross current configuration 130 and concurrent configuration 132, each having angled wall segments 128. The angled nature of the wall segments increases the likelihood of the wall segments intersecting or stacking atop one another when the laminae are stacked. Slight movement of one layer relative to the other in either the x or y direction will still support the membrane at the intersection of the wall segments.

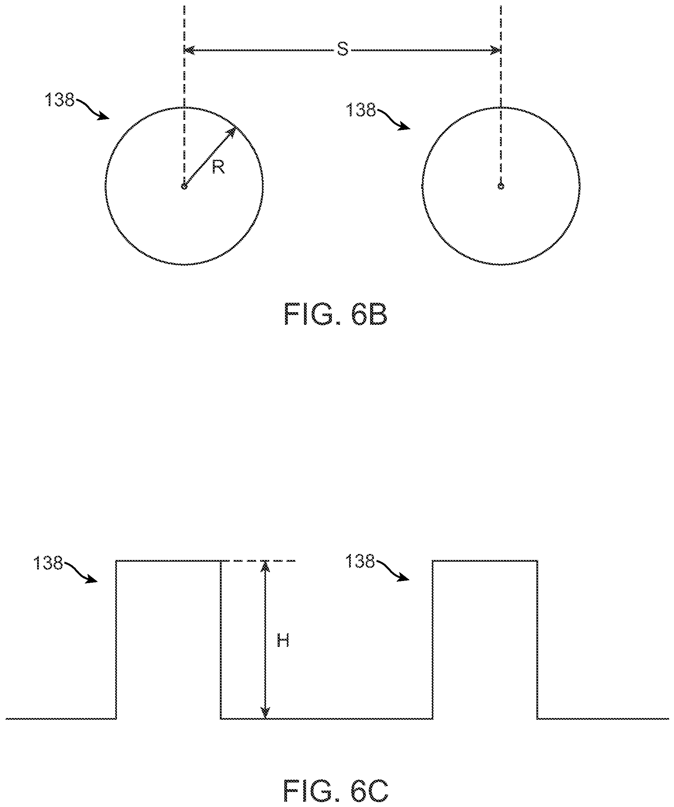

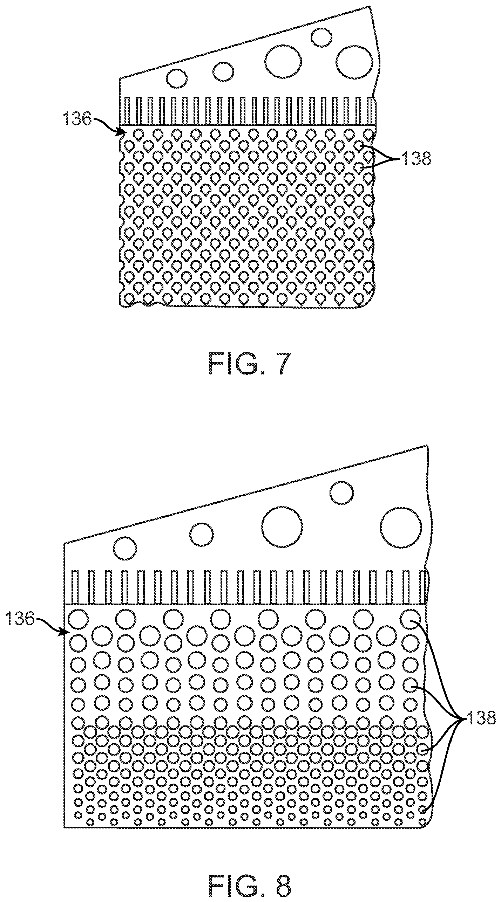

FIG. 6A discloses a laminate 134 having a flow field 136 wherein the support structures comprise plural cylindrical support posts 138. Similar to the wall segments 128 (FIG.3), the plural support posts 138 increase the surface area of the transfer layer (not shown) exposed for transfer. Additionally, fluid is not confined to narrow channels as in microchannels 106 (FIG. 2), thereby allowing fluid to traverse laterally around flow occlusions such as air bubbles or contaminant. FIG. 6B shows a top view of a pair of support structures comprised of the cylindrical support posts 138. FIG. 6C shows a schematic side view of a pair of cylindrical support posts 138. The dimensions of the cylindrical support posts 138 may vary as may the relative spacing between adjacent cylindrical support posts 138 to provide the flow field with desired flow characteristics. For example, the radius R of each support post 138 may be predetermined, as may the distance S between adjacent support posts 138. The height H of each support post may also vary. The support posts 138 may be prisms with circular bases, such as a cylinder. However the bases of the prism may be also of a rectangle, triangle, ellipse, polygon, or tear-drop shape. For instance, FIG. 7 discloses a flow field 136 having support structures formed of tear-drop shaped support posts 138. This embodiment is configured for streamlining fluid flow through the flow field 136.

In an embodiment, the size of the support posts is the minimum possible-measured in diameter for cylindrical posts, width of rectangular posts, or twice the average distance to the geometric center for irregular shapes-without puncturing the transfer layer and large enough to allow alignment of posts of adjacent layers. The support structures are typically greater that zero µm and less than 1000µm. More typically the support structures are greater than zero µm and less than 500 µm, such as about 100 µm to about 400 µm. A person of ordinary skill in the art will recognize that the desired shape and size of the support structures will depend on various factors such as the transfer layer material and thickness, the fluids involved, manufacturing alignment tolerances, and transfer layer efficiency.

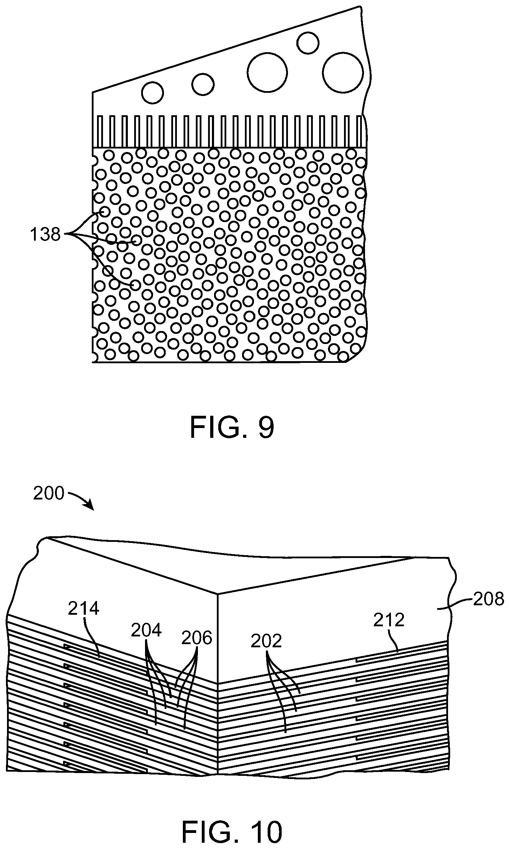

The flow field 136 defines an array of support structures 138 having gradient densities and varying sizes as shown in FIG. 8. One end of the flow field 136 has larger and sparse support structures 138, gradually decreasing in size and increasing in density approaching the opposite end. Additionally some or all of the flow field may be treated with a surface treatment to enhance flow dynamics. For instance the surface may be treated to render it hydrophilic to reduce air entrapment. Alternatively, it may be treated selectively to be hydrophobic in a low support-density area and hydrophilic in a high support-density area, encouraging entrapped air to move through the sparse area to the dense area where wicking and mechanical forces can force the gas out. The support structures 138 may not be randomly distributed throughout the flow field subject to design constraints as shown in FIG. 9.

The distance S between the support structures 138 may be the widest possible considering operating parameters and construction requirements, such as to substantially prevent the transfer layer 110 (FIG.1) from sagging into the flow field 136. The actual width will vary depending on certain factors, such as the rigidity of the transfer layer 110 (FIG. 1) and the operating pressure differential across the transfer layer. Typical widths are between 100 µm and 500 µm, and more typically between about 200 µm and about 400 µm.

Microchannel or flow field depth creates a transfer efficiency advantage. Micron scale dimensions reduce mass transfer limitations by reducing diffusion or conduction lengths through the bulk fluid, thereby increasing the mass rate per unit area of transfer layer 110 (FIG.1), consequently increasing efficiency and reducing device size. Microchannel or flow field depth is typically greater that zero and less than 1000 µm. More typically the depth is greater that zero and less than 400 µm. Even more typically, the depth is greater than zero and less than about 100 µm, such as from about 10 µm to 90 µm.

### 2. Mass Transfer Devices

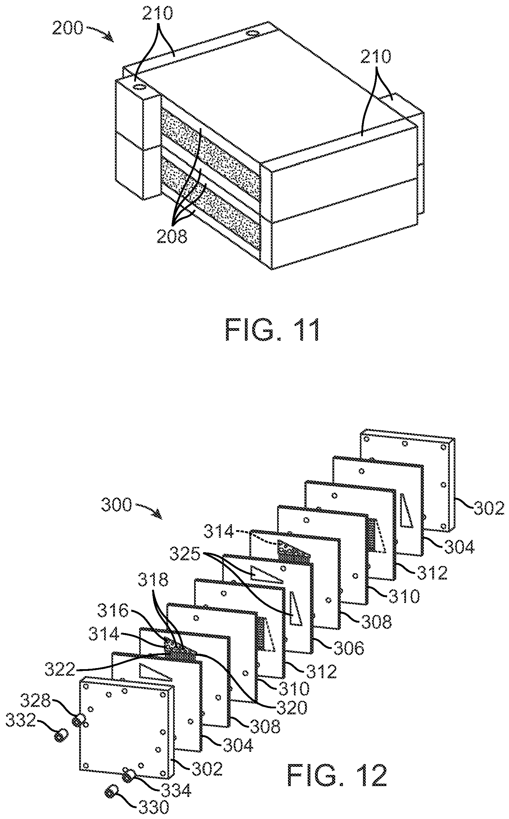

Referring to FIGS. 10 and 11, assembled mass transfer device 200 is comprised of laminae 202, laminae 204, and transfer layers 206. Compression plates 208 are provided to apply pressure to the layered plates 202 and 204 and transfer layers 206 to afford substantially sealed fluid microchannels or flow fields. Compression plates 208 apply pressure using, for example, fasteners connecting the compression plates or by placing device 200 in a clamping mechanism. A person of ordinary skill in the art will recognize that various additional methods of applying force to the compression plates exist in the art. Fluidic headers 210 are operably connected to mass-transfer device 200, and are fluidly connected to microchannel inlets 212 and microchannel outlets 214, for delivering fluids to the internal microchannels 106 and 112 (FIG. 1). FIG. 11 shows two micro fluidic transfer devices arranged in parallel, however a person of ordinary skill in the art will recognize that any number of devices may be configured in parallel, series, or both.

The compression plates 208 may be made from any material with sufficient rigidity to evenly compress the laminae 202 and 204 and transfer layers 206. Suitable materials include, without limitation, polymer, metals, ceramic, or composites. An exemplary material may be, for instance, acrylic. However, a person of ordinary skill in the art will recognize that the compression plate material and its thickness may depend on various factors, for instance, the number of layers in the stack, the required shape to affect a seal, and the operating temperatures. The compression plates 208 may be flat or may have a curved face, such as a convex face, having a curvature suitable for preferably evenly distributing pressure through the device 200.

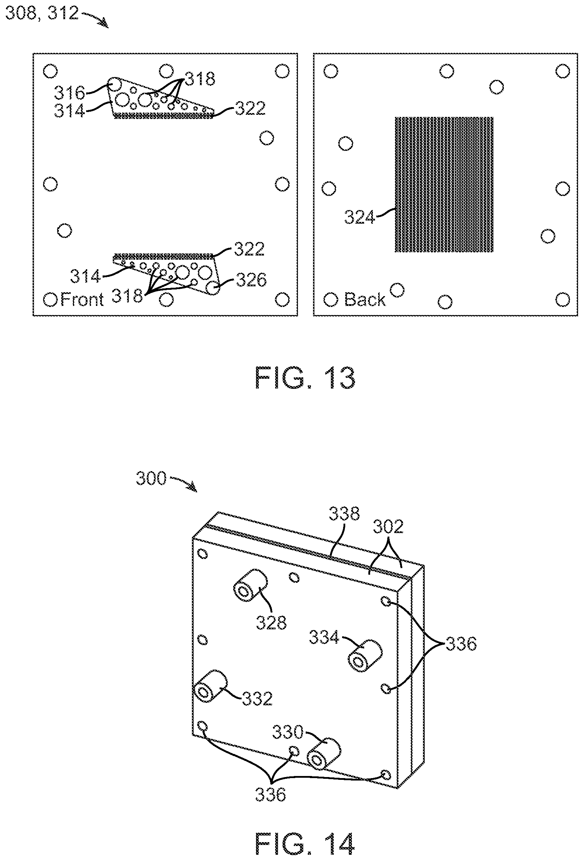

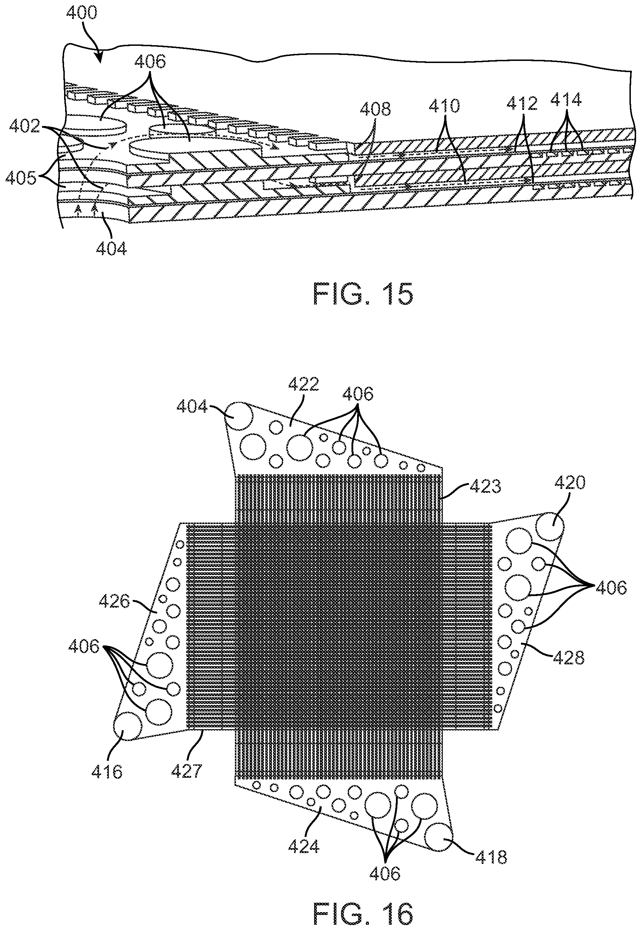

FIG. 12 shows an assembly view of one embodiment of a microfluidic transfer device 300. Mass transfer device 300 comprises a sequenced stack of lamina held between compression plates 302. The sequenced stack comprises head gaskets 304, and repeating subunits separated by gaskets 306. The repeating subunits comprise, in order, a first lamina 308, a transfer layer 310, and a second lamina 312. The number of subunits will depend on the application and the volumetric throughput and transfer capacity required. Additionally, devices may be connected in parallel as shown in FIG. 11. Laminae 308 and 312 are substantially similar in design. Referring to FIG. 13, laminae 308 and 312 have fluid headers 314, fluid inlet 316, support structures 318, vias 322, microchannels 324 (or flow fields) located on the opposite side, and fluid outlet 326. This can be seen in more detail in the discussion of FIG. 16 below. Referring again to FIG. 12, the gaskets 304 and 306 have cutouts 325 so that the gasket does not cover the fluid headers 314, preventing collapse of the gasket material into the header and impeding fluid flow. The support structures 318 may transfer compression force through the stack to facilitate compression sealing throughout the stack and prevent the adjacent lamina from collapsing into the header. The support structures may also prevent the transfer membrane from blocking fluid flow in and through the header. Operatively connected to compression plate 302 are fluid connectors 328, 330, 332, and 334.

FIG. 14 shows a perspective view of assembled micro fluidic transfer device 300. The compression plates 302 have apertures 336 for receiving fasteners for coupling together and compressing the stack 338. A first fluid enters the device 300 through fluid connector 328 and exits the device through fluid connector 330. A second fluid enters the device 300 through fluid connector 332 and exits the device through fluid connector 334.

FIG. 15 provides a detailed view of the internal flow path of two subunits of stack 400. Fluid flow paths 402 show a first fluid entering through fluid inlet 404. Fluid inlet 404 is a through-hole which fluidly connects the first fluid to subunits in the stack. Fluid enters the headers 405, flows around the support structures 406 through the vias 408 to microchannels 410 or flow fields where it contacts transfer layers 412. Transfer layers 412 operatively connect microchannels 410 or flow fields containing the first fluid and microchannels 414 or flow fields containing the second fluid to allow transfer of heat or select substances in the fluids. For instance a mass transfer layer, e.g. a membrane, may allow for membrane permeable components of the first and second fluid to transfer across the membrane from one fluid to the other.

FIG. 16 is a schematic view of the fluid flow patterns of both fluids juxtaposed. Fluid inlet 404 provides the first fluid to inlet header 422, where it flows around support structures 406 to microchannels 423, and is collected at the other end of the microchannels in outlet header 424, then exits through fluid outlet 418. If the embodiment of FIG. 16 included a flow field rather than microchannels 423, then the fluid would flow from the inlet header 422, through the flow field, and toward the outlet header 424. As the fluid flows through the flow field, it would flow around the various support structures positioned within the flow field.

The second fluid enters through fluid inlet 416 into inlet header 426, where it is directed to microchannels 427 or flow field, and is collected in outlet header 428 and exits through outlet 420. FIG. 16 discloses a device having the first and the second fluid flowing orthogonal to each other; however a person of ordinary skill in the art will recognize that one may configure this device for concurrent, countercurrent, or crosscurrent flow.

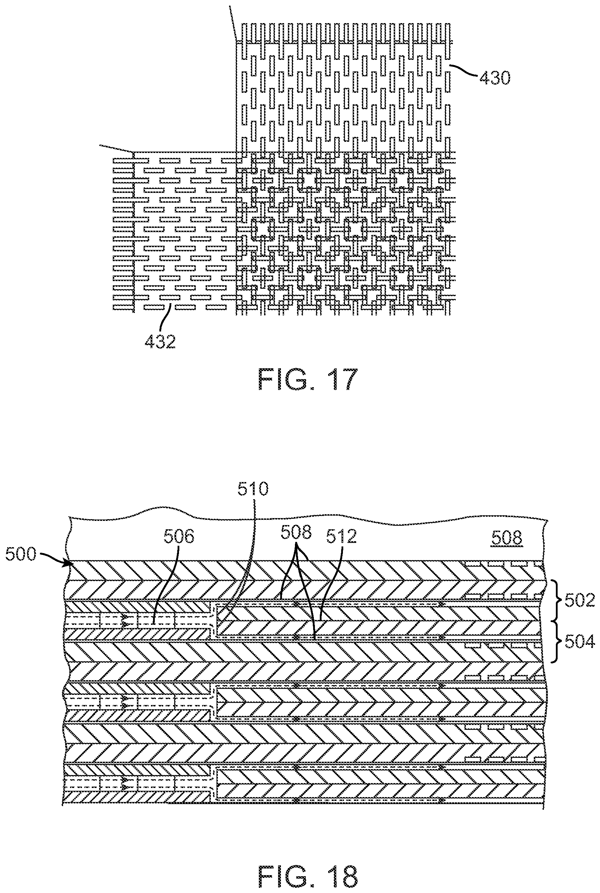

FIG. 17 discloses the juxtaposition of adjacent layers of another embodiment utilizing flow fields 430 and 432 rather than plural parallel microchannels.

In an embodiment, the mass transfer device is a dialyzer such that the first fluid is blood and the second fluid is dialysate. The blood enters the fluid inlet 404and flows to the inlet header 422. The blood then flows into the flow field or microchannels toward the outlet header 424, then exits through fluid outlet 418. The dialysate enters the dialyzer through the fluid inlet 416 and flows into the inlet header 426, where it is directed to microchannels 427 or flow field, and is collected in outlet header 428 and exits through outlet 420. As the blood and dialysate flow through their respective flow fields, solutes diffuse across the mass transfer layer. A pressure gradient may be formed between the respective flow fields in order to achieve hemodiafiltration of the blood where fluid periodically passes from the dialysate into the blood and/or from the blood into the dialysate, thereby transferring molecules by means of convective solute movement that otherwise would be slow to cross the membrane barrier by diffusion alone. Ultrafiltration is a process in dialysis where fluid is caused to move across a dialyzer membrane via diffusion from the blood into the dialysate for the purpose of removing excess fluid from the patient's blood stream. Along with water, some solutes are also drawn across the membrane via convection rather than diffusion. Ultrafiltration is a result of a pressure differential between the blood compartment and the dialysate compartment where fluid will move from a higher pressure to a lower pressure.

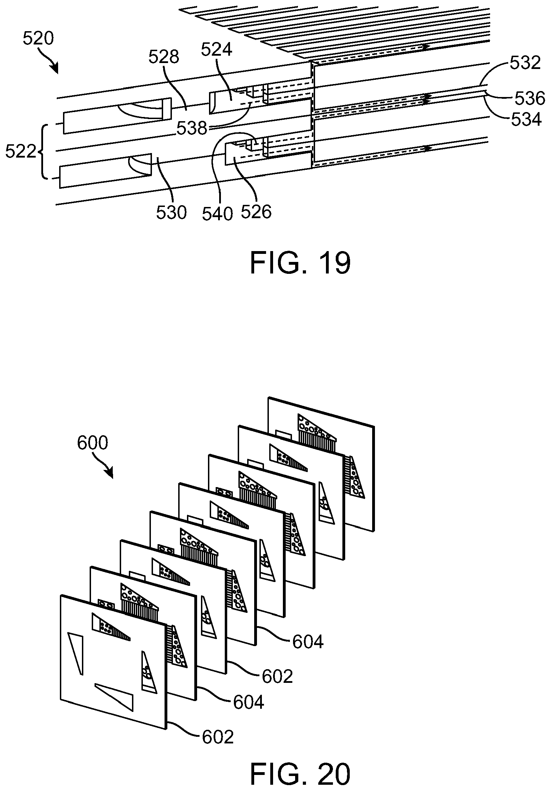

FIG. 18 discloses an embodiment of the device 500 having alternating mirror image subunits 502 and 504. This embodiment creates combined fluid header 506, which directs fluid to microchannels 508 (or flow fields) through vias 510. The subunits 502 and 504 are separated by a gasket 512 with a cutout for header 506. This arrangement reduces the fluid cross contamination relative to embodiments having headers with dissimilar fluids facing each other. Moreover, arranging the subunits 502 and 506 in this manner allows for a single, simplified gasket design compared to the two gasket designs shown as 304 and 306 in FIG. 12. This embodiment may be configured for cross flow as shown in FIG 18 or for concurrent or counter current flow as shown in FIG. 19.

Referring to FIG. 19, device 520 comprises subunit 522 having combined headers 524 and 526. Laminae 528 and 530 comprise parallel microchannels 532 and 534 (or flow fields) separated by transfer layer 536. Parallel microchannels 532 and 534 allow concurrent flow paths 538 and 540. Alternatively, reversing the direction of either flow path 538 or 540 will achieve countercurrent flow.

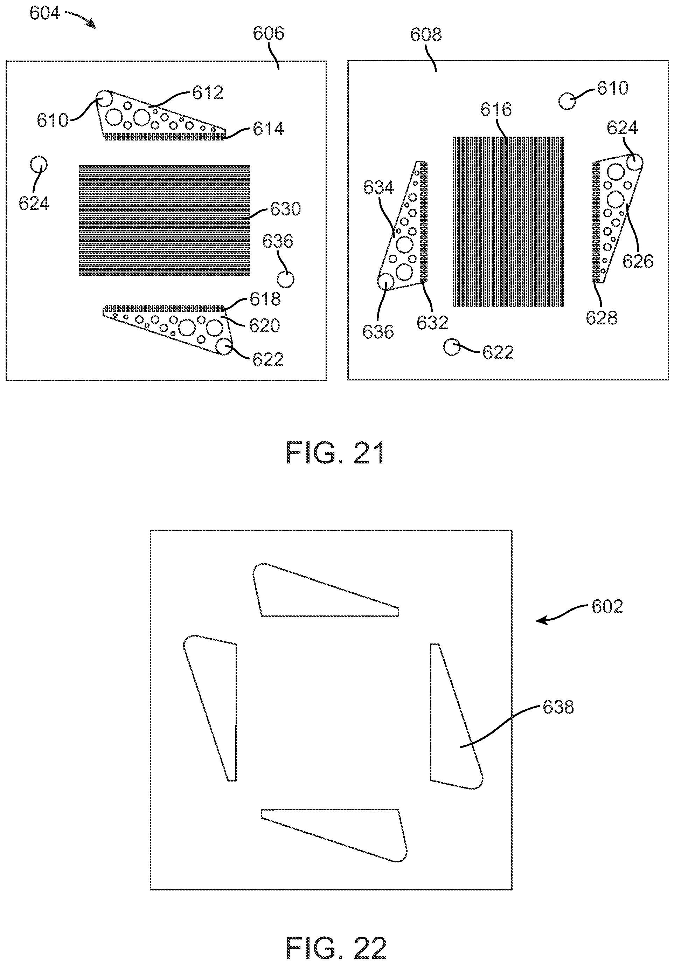

In yet another embodiment, the need for gaskets between subunits is eliminated entirely. FIG. 20 discloses a partial assembly view of a mass transfer device 600 where the laminae 604 have microchannels (or flow fields) and headers on both sides. This configuration allows the device 600 to be assembled as alternate layers of identical transfer layers 602 and laminae 604. FIG. 21 is a plan view of the front 606 and back 608 of the lamina 604. The lamina front 606 has a first fluid inlet 610 fluidly connected to first fluid inlet header 612. First fluid inlet header 612 directs fluid through via 614 to first fluid microchannels 616 (or flow field) on lamina back 608. The microchannels 616 direct fluid to via 618 which fluidly connects the microchannels with first fluid outlet header 620 on lamina front 606, where the first fluid exits by first fluid outlet 622. Similarly, the lamina back 608 has second fluid inlet 624 which fluidly connects to second fluid inlet header 626. Second fluid inlet header 626 is fluidly connected to via 628 which fluidly connects the second fluid inlet header to the second fluid microchannels 630 (or flow field) on the lamina front 606. The second fluid microchannels 630 direct the fluid to via 632 which fluidly connects the second fluid microchannels and the second fluid outlet header 634, which is fluidly connected to the second fluid outlet 636.

FIG. 22 discloses the transfer layer 602 used in microfluidic transfer device 600 (FIG. 20). The transfer layer 602 has four cutouts 638 associated with the locations of the fluid headers 612, 620, 626, and 634 on plate 604 (FIG. 21). While the double sided lamina 604 allows a device with nearly half the number of lamina compared to previously disclosed embodiments, compression alone may not adequately seal the transfer layer between the headers and microchannels located on the same side of the lamina.

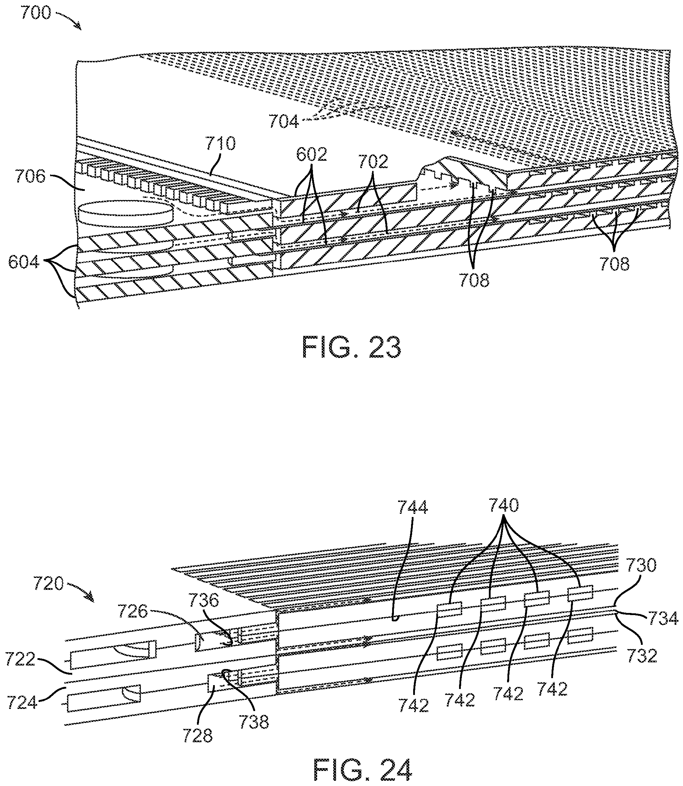

FIG. 23 shows a detailed view of a microfluidic transfer device 700 employing double sided lamina 604. The first fluid flows from the headers 706 through lamina 604 to microchannels 702 (or flow field). Similarly the second fluid flows from a header, not shown, on lamina 604 to microchannels 704 (or flow field) located on the same side of the plate as header 706. Because the transfer layer 602 is compressed against adjacent layers by microchannel dividers 708 rather than a solid surface, fluid could leak under the transfer layer allowing fluid from header 706 to enter microchannel 704. Transfer layer bond 710 prevents this. Adhesives or laser welding could create the transfer layer bond 710, however a person of skill in the art will recognize that one may employ other methods to create the bond. Such methods include but are not limited to RF welding, ultrasonic welding, and thermal welding.

While FIG. 23 discloses a double-sided device with crosscurrent flow, it is also possible to configure a double-sided device with concurrent or countercurrent flow. For example, FIG. 24 illustrates device 720 having double-sided laminae 722 and 724 arranged to provide combined headers 726 and 728. Microchannels 730 and 732 are parallel and are separated by transfer layer 734, allowing concurrent flow paths 736 and 738. Likewise microchannels 740 and 742 are parallel to each other and are separated by transfer layer 744, allowing concurrent flow paths not shown. A person of ordinary skill in the art will recognize that this embodiment also allows countercurrent flow.

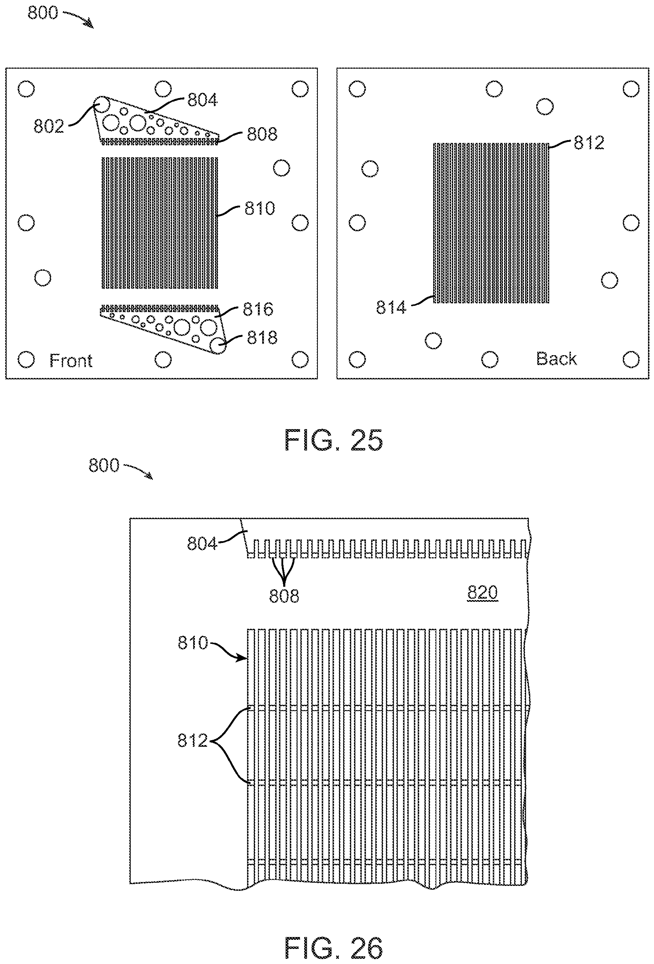

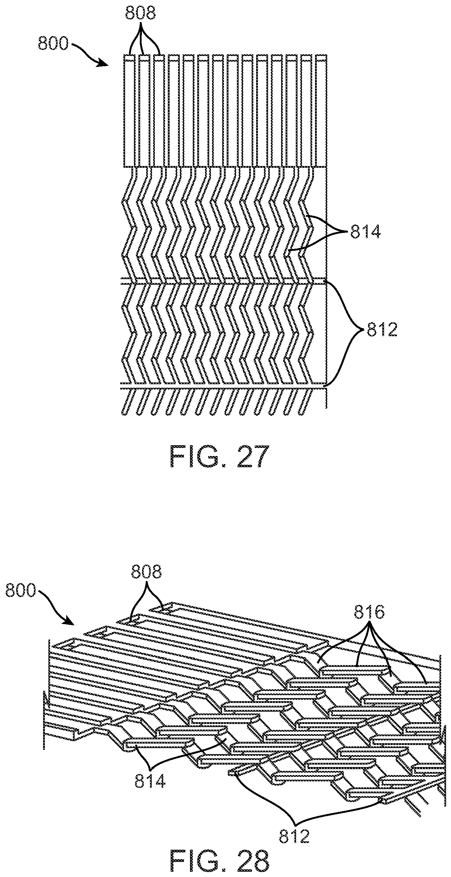

One embodiment of the microfluidic transfer device employs microchannels that are cut through the entire lamina thickness. FIG. 25 is a plan view of a through-cut lamina 800. Lamina 800 has fluid inlet 802, fluidly connected to inlet header 804. Inlet header 804 is fluidly connected to via 808. Through-cut microchannels 810 are fluidly connected to vias 808 by way of microchannels 812. Microchannels 814 fluidly connect through-cut microchannels 810 with outlet header 816, which directs fluid flow to outlet 818. With the microchannels cut through the entire lamina thickness, the microchannel dividers may need structural support. FIG. 26 shows lamina 800 having through-cut microchannels 810 supported by partial thickness dividers 812. To afford a robust compression seal, lamina 800 has a compression seal face 820 for compressing the transfer layer against the adjacent layer. Another embodiment of a through-cut microchannel lamina is shown in FIG. 27.

FIG. 27 shows a plan view of lamina 800 having microchannel dividers forming a herringbone pattern. Referring to FIG. 28, microchannel dividers 814 comprise plural partial thickness wall segments 816 arranged in a herringbone pattern. Partial thickness wall segments 816 alternate in the herringbone pattern such that adjacent wall segments are flush with opposite sides of the lamina 800. This design increases device efficiency by exposing a greater surface area of the transfer layer (not shown). The partial thickness wall segments 816 may essentially form a flow field rather than microchannels, as the partial thickness wall segments 816 do not necessarily constrain the fluid flow into a single channel.

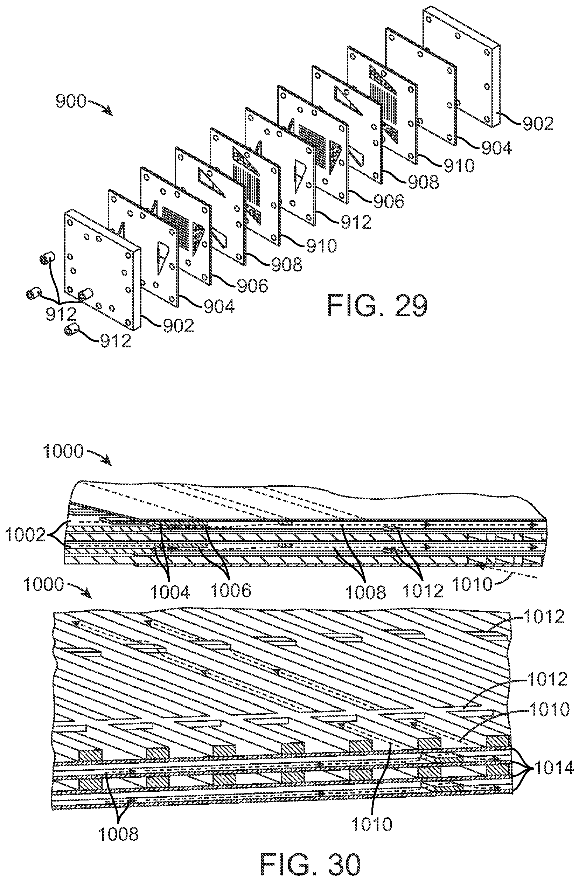

FIG. 29 shows an assembly view of microfluidic transfer device 900 using through-cut laminae 906. Compression plates 902, operatively connected to gaskets 904, hold and compress repeating subunits comprising, in order, first fluid lamina 906, transfer layer 908, and second fluid lamina 910. The subunits are separated by transfer layers 912. One advantage of this embodiment is the increased transfer layer exposure per microchannel. Since the through-cut microchannels are bound on two sides by transfer layers 908 and 912, which operatively connect them to adjacent plates, the transfer layer surface area per lamina is almost doubled. This allows for fewer layers, and allows reduced costs and smaller devices.

FIG. 30 provides a detail of the fluid flow path 1000. Fluid enters the inlet header 1002, which directs the fluid to via 1004. The fluid travels through via 1004 to microchannel 1006, then to through-cut microchannel 1008. Through-cut microchannel 1008 is oriented orthogonal to through-cut microchannel 1010. Through-cut microchannels 1008 and 1010 have partial thickness dividers 1012 for structural support. Additionally, dividers 1012 provide mixing without substantially impeding fluid flow. Transfer layers 1014 separate and operably connect through-cut microchannels 1008 and 1010 to afford heat or mass transfer from one fluid to another.

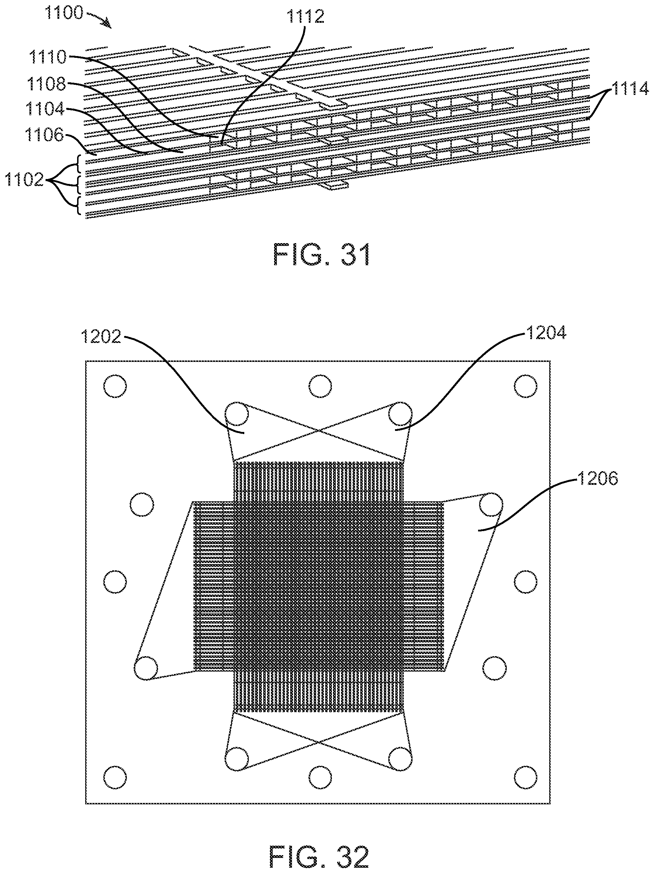

FIG. 31 discloses a detail of a through cut device 1100 having both concurrent and cross current flow. Device 1100 comprises plural subunits 1102. Subunit 1102 comprises a transfer layer 1104 between a first lamina 1106 and a second lamina 1108. Laminae 1106 and 1108 have through-cut microchannels 1110 and 1112, respectively. Microchannels 1110 and 1112 are parallel to each other and orthogonal to microchannels of adjacent subunits 1102. The subunits 1102 are separated by transfer layers 1114. Consequently, subunits 1102 have concurrent or countercurrent flow between laminae 1106 and 1108 within subunit 1102, and crosscurrent flow between subunits.

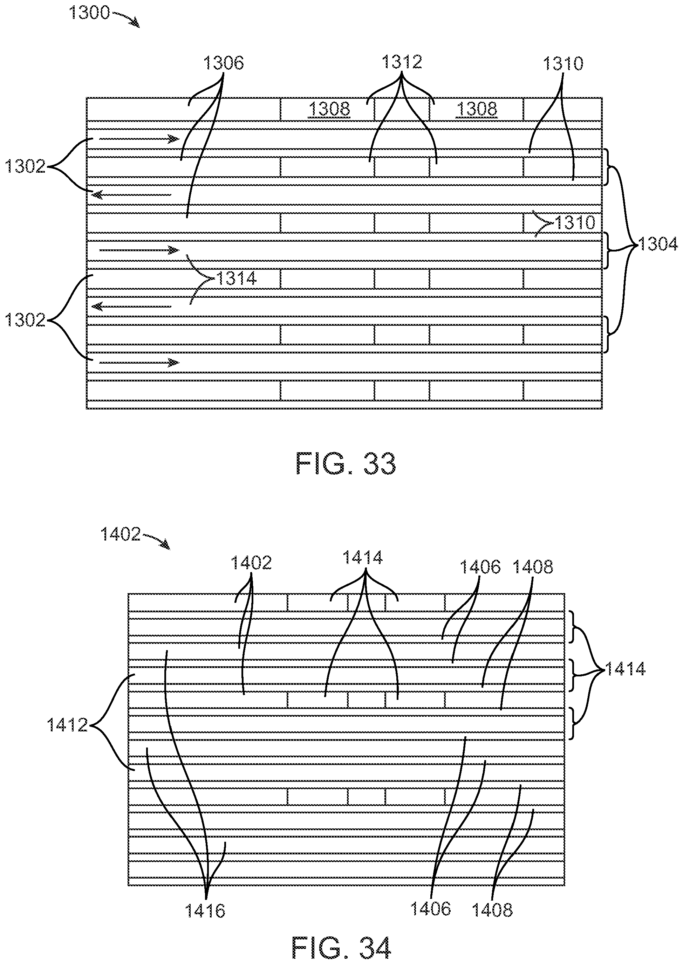

The disclosed device may utilize fluid membranes. FIG. 32 discloses a plan view of the juxtaposition of the process fluid flow paths 1202 and 1204 and the fluid membrane channels 1206. Fluid flow paths 1202 and 1204 are substantially parallel to each other and substantially orthogonal to the fluid membrane channels 1206. Referring now to FIG. 33, fluid membrane device 1300 comprises through-cut laminae separated by fluid membranes 1304. Fluid membranes 1304 comprises through-cut lamina 1306 containing fluid 1308 and membrane supports 1310. Through-cut lamina 1308 has microchannels 1312 substantially orthogonal to microchannels 1314 of through-cut laminae 1302. A person of ordinary skill in the art will recognize that the membrane supports may be any material suitable for liquid membrane applications. For example and without limitation, a microporous polyethylene film may be used as a membrane support. A person of ordinary skill in the art will recognize that the need for, composition and positioning of membranes support will depend on, for example, the fluid used in the fluid membrane, the process fluids, and the operating temperatures and pressures.

The disclosed devise may also be configured as a fuel cell. FIG. 34 discloses a fuel cell device 1400 comprising plural through-cut lamina 1402 separated by a transfer layer 1404 comprising a cathode 1406, an anode 1408, and a polymer electrolyte membrane 1412 therebetween. The device of FIG. 34 may contain, for instance, hydrogen in microchannels 1414, and oxygen in microchannels 1416. Transfer layers 1404 are oriented such that the anode 1408 is adjacent to the microchannels 1414 and the cathode is adjacent to the microchannels 1416. A person of ordinary skill in the art will recognize that this device may be used with any fuel cell and the transfer layer configuration will depend on, for instance, the fuels used and the operating temperature and pressure. A person of ordinary skill in the art will also recognize that the device may also be configured for concurrent or countercurrent flow.

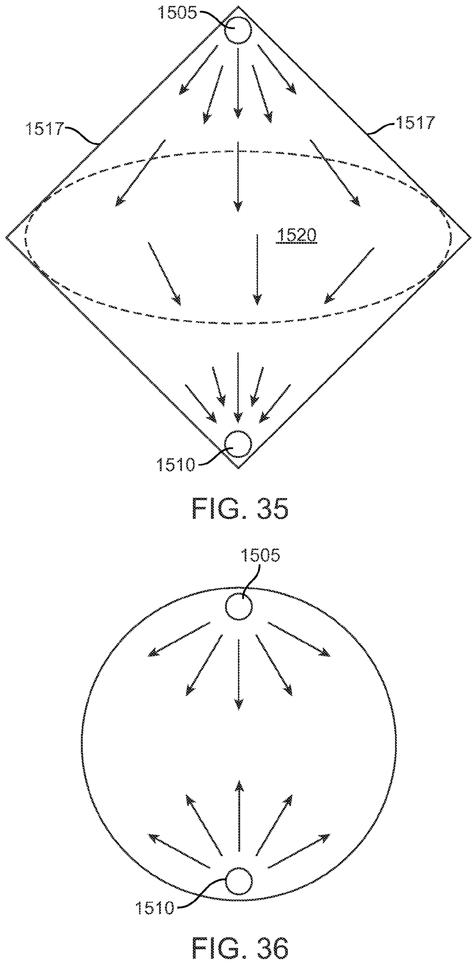

FIG. 35 shows a plan view of an embodiment of a lamina of a flow field dialyzer without header regions. In this embodiment, the flow field has a polygonal shape with an inlet 1505 positioned at an upper point of the flow field and an outlet 1510 positioned at a lower point of the flow field. A plurality of support structures, such as pins, are located within the flow field. For clarity of illustration, the support structures are not shown in the flow field of FIG. 35. The configuration of the support structures within the flow field may vary as described above with reference to Figures 3-9.

The flow field is defined by opposed walls 1517 with a space therebetween for fluid flow. The walls 1517 diverge from the inlet 1505 such that the flow field has relatively small transverse size in the region of the inlet 1505 and a widened transverse size in a central region 1520. The central region 1520 is approximately represented with an oval shape in FIG. 35, although the shape of the central region may vary. From the central region 1520, the walls 1517 converge toward the outlet 1510 such that the flow field has a smaller transverse size at the outlet 1510 relative to the central region 1510. The inlet 1505 supplies fluid into the flow field without any particular flow region for the fluid to attain a relatively even distribution before entering the flow field.

The relatively constrained size at the inlet 1505 relative to the central region 1520 results in a pressure differential between fluid flowing at the inlet relative to fluid flowing at the central region 1520. That is, the pressure drops as the fluid flows into the widened central region. The pressure then rises as the fluid flows toward the smaller region of the outlet 1510. This results in an increase in fluid velocity as the fluid flows from the inlet 1505 toward the central region 1520, and then a decrease in velocity as the fluid flows from the central region 1520 toward the outlet 1510. The flow field may vary in shape and can have any of a variety of shapes that achieve the size differential between the regions of the inlet/outlet and the central region. For example, FIG. 36 shows a circular flow field that achieves size differentials between the regions of the inlet/outlet and the central region. Other shapes are possible, such as oval, diamond, etc.

In such an embodiment, no header may be required as a result of the flow field itself acting as its own header region and attaining a relatively even flow distribution simply through the effect of the pressure drop between the relatively higher pressure, higher fluid velocity region associated with the incoming fluid stream immediately adjacent the inlet 1505, and the relatively lower pressure, lower velocity region 1520 towards the center region 1520 of the flow field, combined with the various supports structures such as pins that the fluid impinges upon and flows around to create an even flow distribution. As more fluid enters the flow field through the inlet 1505, the fluid already in the flow field is pushed towards and out of the outlet 1510. Moreover, the reduction in fluid velocity as the fluid flows into the central region 1520 results in an increase in the residence time for fluid in the flow field. The increased residence time may result in an increased amount of diffusion across the dialyzer membrane and increased efficiency of the dialyzer.

In an embodiment, the pins 1512 are arranged in a series of rows such that the pins essentially form channels through the flow field. Using known techniques, channels of a certain depth between the rows of pins can be achieved as follows. First a master lamina may be created, for example, by machining a suitable material, such as aluminum, to the desired dimensions or by laser etching a sheet of suitable material, such as a polyimide sheet. In an embodiment, a sufficient amount of laminae are used to form a rectangular flow field having dimensions of about 10 centimeters by about 10 centimeters although variations are possible. An embossing master is then created from the master lamina either by embossing a polyetherimide sheet with the previously created master, or by a combination of laser etching and embossing with the previously created master. Finally, each lamina is created from the embossing master. It should be appreciated that variations are possible in the method of manufacture.

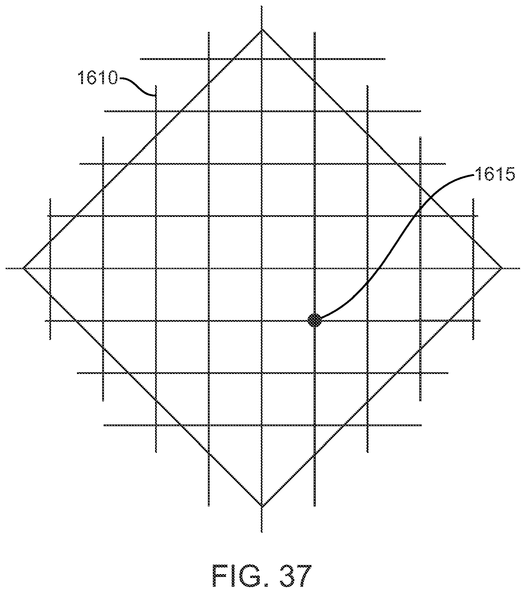

In creating the master using laser etching, the paths of the laser beams cut pathways of relatively even depth into the substrate. This is represented schematically in FIG. 37, where the lines 1610 represent successive pathways of laser beams that form the channels. A channel of relatively even depth into the lamina is formed along the length of each laser pathway. However, where the laser pathways cross one another, such as at junction 1615 , the lamina is cut about twice as deep as where the laser pathways do not cross. The increased depth at the junctions 1615 is at least partially a result of the laser energy multiplying where the two lasers cross one another. This results in an undulating path for each channel wherein each channel has a relatively uniform depth along a portion of its length and increased depth at the junctions 1615.

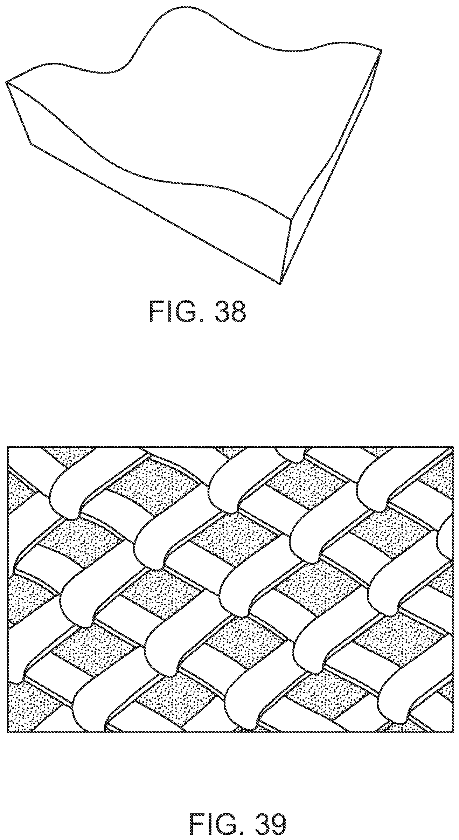

FIG. 38 shows an enlarged view of a portion of lamina where the laser-formed channels intersect and have this type of undulating-floor channel resulting from a laser-etched cut. FIG. 39 shows an enlarged view of the lamina surface showing the undulating channels and pins formed between the channels. The embodiment shown in FIG. 39 has raised surfaces that are generally flat on the sides and top. In another embodiment, the raised surfaces are rounded on the sides and top. The undulating channel pathway floor results in more mixing conditions in the flow than would otherwise be achieved with a pathway floor between all pins of relatively equal depth, such as is typically creating when machining aluminum, for example. That is, the undulating channel pathway floor results in localized variations in flow velocity and flow direction in each region of increased depth. This causes localized mixing of the fluid as it flows along the regions of increased depth. The mixing tends to increase the efficiency of the device by repeatedly bringing fresh dialysate closer to the surface of the transfer membrane.

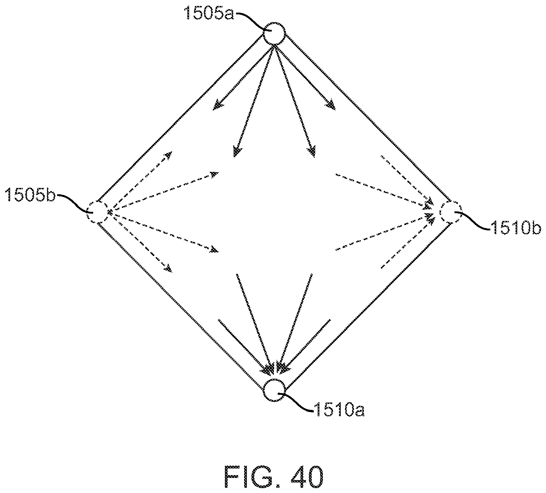

FIG. 40 shows an embodiment where alternating symmetrical laminae are stacked in a cross-current manner for separation of the inlets 1505a and outlets 1510a of the laminae handling the fluid to be dialyzed, for example, from the inlets 1505b and outlets 1510b of the interleaved laminae handling the dialysate. For such an embodiment, each lamina may be substantially symmetrical about a central axis, such as square- or circle-shaped, so that even stacking may be achieved. Almost any degree of counter-current or cross-current or con-current flow with a headerless flow field and appropriately located inlets and outlets may be configured and would fall within the scope of the present invention.

To determine the feasibility of using the disclosed device for hemodialysis, one-, three-, and five-layer microchannel-based devices and a single-layer flow-field device were fabricated. The microchannel-based device contained microchannels that were 100 µm deep and 400 µm wide with 200 µm wide dividers. There are 51 channels in the array, giving a relatively small membrane transfer area of 4.2 cm² per layer (or transfer unit). The flow-field design had 6.3 cm² of membrane transfer area with a flow field depth of 60 µm. The laminae were prepared and patterned using a hot emboss technique. All devices were configured for cross flow and sealed using compression. The transfer layers were AN69 flat sheet membranes available from Gambro Medical.

Flow rates of fluids across the various microfluidic embodiments disclosed depend on the flow rate across an individual lamina and the number of lamina in a stack. In a microfluidic device that is being used for dialysis within a dialysis system, the flow rate across the microfluidic dialyzer may be substantially matched with the flow rate of dialysate being produced up-stream of the dialyzer, such as disclosed in U.S. Patent Application entitled "Dialysis System with Ultrapurification Control," filed on June 7, 2010, and naming James R. Curtis, Ladislaus F. Nonn, and Julie Wrazel is incorporated herein by reference in its entirety. In this manner flow rates of up to 1000 ml/min may be achieved, though lower flow rates, such as 100 ml/min across either side of the membrane may be preferred for dialysis applications outside of the clinical setting, such as home or nocturnal dialysis. U.S. Patent Application entitled "Fluid Purification System," filed on June 7, 2010, naming Richard B. Peterson, James R. Curtis, Hailei Wang, Robbie Ingram-Gobel, Luke W. Fisher and Anna E. Garrison, and "Dialysis System," filed on June 7, 2010, naming Julie Wrazel, James R. Curtis, Ladislaus F. Nonn, Richard B. Peterson, Hailei Wang, Robbie Ingram-Govel, Luke W. Fisher, Anna B. Garrision, M. Kevin Drost, Goran Jovanovic, Todd Miller, Bruce Johnson and Alana Warner-Tuhy, also are incorporated herein by reference in their entirety.

### C. Making Microfluidic Transfer Devices

Devices disclosed herein may be produced by a many of the techniques involved in a fabrication approach known as microlamination. Microlamination methods are described in several patents and pending applications commonly assigned to Oregon State University, including U.S. Patents, Nos. 6,793,831, 6,672,502, and U.S. Publication, Nos. 2007/0029365, entitled High Volume Microlamination Production of Mecs Devices, and 2008/0108122, entitled Microchemical Nanofactories, all of which are incorporated herein by reference.

Microlamination consists of patterning and bonding thin layers of material, called laminae, to generate a monolithic device with embedded features. Microlamination typically involves at least three levels of production technology: 1) lamina patterning, 2) laminae registration, and 3) laminae bonding. Thus, the method of the present invention for making devices comprises providing plural laminae, registering the laminae, and bonding the laminae. Laminae bonding is not required for all disclosed embodiments, as the registered lamina are held between compression plates affording a compression seal. As yet another alternative, certain embodiments may have at least some laminae bonded together in combination with compression. The method also may include dissociating components (i.e., substructures from structures) to make the device. Component dissociation can be performed prior to, subsequent to, or simultaneously with bonding the laminae.

In one aspect of the invention, laminae are formed from a variety of materials, particularly metals; alloys, including intermetallic metals and super alloys; polymeric materials, including solely by way of example and without limitation, polycarbonate, polyethylene terephthalate (PET), polyether imide (PEI), poly(methyl methacrylate) (PMMA), and halogenated polyethylene such as poly(tetrafluoroethylene) (PTFE); ceramics; and combinations of such materials. The proper selection of a material for a particular application will be determined by various factors, such as the physical properties of the metal or metal alloy and cost. Examples of metals and alloys particularly useful for metal microlamination include stainless steels, copper, titanium, nickel, and aluminum. Laminae useful for the microlamination method of the present invention can have a variety of sizes. Generally, the laminae have thicknesses of from about 25 µm to about 1000 µm thick, preferably from about 25 µm to about 500 µm thick, and even more preferably from about 25 µm to 250 µm thick. Individual lamina within a stack also can have different thicknesses.

### 1. Lamina Patterns

Lamina patterning may comprise machining or etching a pattern in the lamina. Lamina patterning may also comprise embossing, roll embossing, and/or stamping. The pattern produced depends on the device being made. Without limitation, techniques for machining or etching include laser-beam, electron-beam, ion-beam, electrochemical, electrodischarge, chemical and mechanical material deposition or removal. The lamina can be patterned by combinations of techniques, such as both lithographic and non-lithographic processes. Lithographic processes include micromolding and electroplating methods, such as LIGA, and other net-shape fabrication techniques. Some additional examples of lithographic techniques include chemical micromachining (i.e., wet etching), photochemical machining, through-mask electrochemical micromachining (EMM), plasma etching, as well as deposition techniques, such as chemical vaporization deposition, sputtering, evaporation, and electroplating. Non-lithographic techniques include electrodischarge machining (EDM), mechanical micromachining and laser micromachining (i.e., laser photoablation). Photochemical and electrochemical micromachining likely are preferred for mass-producing devices.

One method for patterning lamina for disclosed device embodiments is microembossing. For instance, certain embodiments of the present disclosure were made using the following techniques. An Obducat Nano Imprint Lithography system was used to transfer microscale patterns from masters to polymeric parts. Master fabrication was accomplished by micromilling masters in metal, such as aluminum. A double transfer process using another material, such as polyether imide (PEI), as the intermediate was also used. A triple transfer process using patterned photoresist as the starting master was also used. The pattern was transferred from the photoresist, typically SU-8, to polydimethylsiloxane (PDMS), then to a thermoset epoxy (e.g., Conapoxy FR-1080) which then was used as the embossing master in the Obducat tool, transferring the pattern to a lower melting temperature polymer, such as polyethylene terephthalate (PET). The SU-8 can be deposited and patterned in multiple layers, allowing creation of precision multiplane masters. These planes can be both above and below the plane with the compression seal, allowing, for example, formation of protruding features such as sealing bosses as well as channels with multiple depths. Laminae also can be embossed on both sides simultaneously used two masters. Alignment techniques such as marks and pins were used during prototyping. It is anticipated that volume production will be accomplished using roll embossing and lamination techniques, also known as conversion processes, that will include automated alignment using vision systems.

Another method used for making disclosed embodiments was photochemical etching of metal laminae, e.g., 316/316L stainless steel. Patterned photoresist was used to mask both the front and back side of the laminae, with different masking patterns for each side. Partial etching from each side created intricate flow channels, including vias from one side to the other and channels open to both sides. Small support structures used to stabilize the channel dividers were also created. Such structures can be used to create segmented channel divider architectures, thereby increasing the active surface area of the transfer layer.

Laser machining was also used to cut vias, inlet and outlet ports, and alignment pin holes in laminae as well as embossing masters. An ESI 5330 with a 355nm wavelength laser was used for laser machining. In volume production a laser may be also used to cut vias and other penetrations. To create the via, the angle of the laser will preferably be non-orthogonal to create a non-orthogonal via, thereby reducing dead volumes in the flow channel. Alternatively, the vias and other penetrations may be created using a stamping operation. The stamping operation may be accomplished as part of the embossing operation through design of appropriate embossing/stamping masters. Non-orthogonal vias in particular are also created by designing appropriate embossing/stamping masters.

Laser micromachining has been accomplished with pulsed or continuous laser action. Machining systems based on Nd:YAG and excimer lasers are typically pulsed, while CO₂ laser systems are continuous. Electro Scientific Industries model 4420 is a typical system for Nd:YAG. This micromachining system uses two degrees of freedom by moving the focused laser flux across a part in a digitally controlled X-Y motion. The cutting action is either thermally or chemically ablative, depending on the material being machined and the wavelength used. The drive mechanism for the Nd:YAG laser may be a digitally controlled servo actuator that provides a resolution of approximately 2 µm. The width of the through cut, however, depends on the diameter of the focused beam.

Laminae also have been machined with CO₂ laser systems. Most of the commercial CO₂ lasers semi-ablate or liquefy the material being cut. A high-velocity gas jet often is used to help remove debris. As with the Nd:YAG systems, the laser (or workpiece) is translated in the X-Y directions to obtain a desired pattern in the material.

An Nd:YAG pulse laser has been used to cut through, for example, 90-µm-thick steel shims. The line widths for these cuts were approximately 35 µm wide, although with steel, some tapering was observed. Some debris and ridging may occur along the edge of the cut on the front side. This material may be removed easily from the surface during lamina preparation, such as by surface polishing.

Laminae also may be patterned using a CO₂ laser. The CO₂ through-cuts were approximately 200 µm wide and also exhibited a slight taper. The width of the CO₂ laser cut was the minimum achievable with the system used. The part may be cleaned in a lamina preparation step using surface polishing to remove debris.

Pulsed Nd:YAG lasers also are capable of micromachining laminae made from polymeric materials, such as laminae made from polyimides. Pulsed Nd:YAG lasers are capable of micromachining these materials with high resolution and no recast debris. Ultraviolet wavelengths appear best for this type of work where chemical ablation apparently is the mechanism involved in removing material. Clean, sharp-edged holes in the 25 - 50 µm diameter range have been produced.

### 2. Lamina Preparation

Depending on the lamina and patterning technique used, lamina patterning may include lamina preparation. The laminae can be prepared by a variety of techniques. For example, surface polishing of a lamina following pattern formation may be beneficial. Moreover, acid etching can be used to remove any oxides from a metal or alloy lamina. Lamina preparation may also include applying an oxide-free coating to some or all of the laminae. An example of this would be electroplating gold onto the lamina to prevent oxidation at ambient conditions.

### 3. Registration

Laminae registration comprises (1) stacking the laminae so that each of the plural lamina in a stack used to make a device is in its proper location within the stack, and (2) placing adjacent laminae with respect to each other so that they are properly aligned as determined by the design of the device. It should be recognized that a variety of methods can be used to properly align laminae, including manually and visually aligning laminae.

The precision to which laminae can be positioned with respect to one another may determine whether a final device will function. The complexity may range from structures such as microchannel arrays, which are tolerant to a certain degree of misalignment, to more sophisticated devices requiring highly precise alignment. A person of ordinary skill in the art will recognize that microchannels on adjacent laminae that are parallel to each other require a greater precision of alignment that embodiments having cross current flow. Several alignment methods can be used to achieve the desired precision. Registration can be accomplished, for example, using an alignment jig that accepts the stack of laminae and aligns each using some embedded feature, e.g., corners and edges, which work best if such features are common to all laminae. Another approach incorporates alignment features, such as holes, into each lamina at the same time other features are being machined. Alignment jigs are then used that incorporate pins that pass through the alignment holes. The edge alignment approach can register laminae to within 10 microns, assuming the laminae edges are accurate to this precision. With alignment pins and a highly accurate lamina machining technique, micron-level positioning is feasible.

Vision systems and thermally assisted lamina registration also can be used as desired. Additional detail concerning thermally assisted lamina registration is provided by Patent Publication No. 2007/0029365, which is incorporated herein by reference. A person of ordinary skill in the art also will recognize that the registration process can be automated.

### 4. Device Manufacture

Laminae bonding comprises bonding at least some of plural laminae one to another to produce a monolithic device (also referred to as a laminate). Laminae bonding can be accomplished by a number of methods including, without limitation, diffusion soldering/bonding, thermal brazing, adhesive bonding, thermal adhesive bonding, curative adhesive bonding, electrostatic bonding, resistance welding, microprojection welding, and combinations thereof. In addition to or as an alternative to bonding the registered lamina, the disclosed device may be assembled between compression plates. However, for some applications, bonding the lamina to the transfer layer may be preferable. Additionally, a bond or weld, such as a laser tack weld, may be used to facilitate assembly during manufacture.

A preferred method of device fabrication involves high through-put, low cost fabrication techniques. Laminae patterning is accomplished using several techniques, including embossing, stamping, and photochemical etching, among others. In one preferred embodiment, assembly is accomplished using roll techniques, such as those used in web processing or conversion industries. Polymer films are roll embossed and stamped, then laminated to form a subassembly. Metal laminae are patterned using photochemical etching. Abrasive waterjet techniques under development now may also be used for patterning metal laminae in the future. The subassemblies are separated, stacked, and assembled in compression frames. The primary sealing method is by compression from an external frame, however, bonding techniques such as laser welding and adhesives may be used for portions of some embodiments. A sealant or sealing method may be applied to the edges to prevent external seepage through the membrane.

### 5. Heat Transfer Operations

In other embodiments, the microfluidic transfer devices disclosed herein can be used in various heat transfer operations. As with the mass transfer devices disclosed herein, heat transfer devices can comprise a stack of plural subunits to scale the device to the desired volumetric capacity. Thermally conductive layers can be incorporated into such devices (e.g., positioned between the subunits) to allow heat to transfer from one fluid to another.

For example, referring to FIG. 1, in a heat transfer embodiment, transfer layer 110 can be a heat transfer layer for allowing heat to transfer from the fluid in microchannel 106 to the fluid in microchannel 112, or visa-versa. In this embodiment, transfer layer 110 can be any material capable of conducting heat from one fluid to another at a sufficient rate for the desired application. Relevant factors include, without limitation, the thermal conductivity of the heat transfer layer 110, the thickness of the heat transfer layer, and the desired rate of heat transfer. Suitable materials include, without limitation, metal, metal alloy, ceramic, polymer, or composites thereof. Suitable metals include, without limitation, iron, copper, aluminum, nickel, titanium, gold, silver, or tin. Copper may be a particularly desirable material.

Similar to the mass transfer devices described herein, the micron scale dimensions of a microfluidic heat transfer device reduces heat transfer limitations by reducing diffusion or conduction lengths through the bulk fluid, thereby increasing the heat transfer rate per unit area of transfer layer 110 (FIG. 1), consequently increasing efficiency and reducing device size.

Disclosed embodiments also may incorporate both heat and mass transfer components. A person of ordinary skill in the art will recognize that a number of configurations are possible and the desired application will dictate optimal configurations.

In view of the many possible embodiments to which the principles of the disclosed device may be applied, it will be recognized that the illustrated embodiments are only preferred examples and should not be construed as limiting the claims to a scope narrower than would be appreciated by a person of ordinary skill in the art. Rather, the scope is defined by the following claims.

## Claims

1. A microfluidic device for performing dialysis, comprising:
a first lamina comprising a first flow field comprising a first set of support structures around which blood flows during operation;
a second lamina comprising a second flow field (136) comprising a second set of support structures (138) around which dialysate flows during operation, wherein each of the support structures (138) in at least one of the first flow field and the second flow field (136) comprises a column having a base with a shape selected from a circle, tear-drop, triangle, ellipse, or polygon, and wherein the support structures in at least one of the first flow field and the second flow field (136) have a gradient of size and/or a gradient of density from one end of the flow field (136) to the opposite end of the flow field (136); and
at least one mass transfer layer interleaved between the first and second flow fields (136), across which layer dialysis of the blood occurs when in operation,
**characterized in that** the support structures (138) in at least one of the first flow field and second flow field (136) have a gradient of size and a gradient of density in the flow field (136), gradually decreasing in size and increasing in density from one end of the flow field (136) to the opposite end of the flow field (136).

2. The device according to claim 1, wherein the device does not comprise a header region, the device further comprises a header region fluidly connected to at least one of the first or second flow fields, or the device further comprises header regions fluidly connected to both the first and second flow fields.

3. The device according to claim 1 wherein the first and second laminae together with the interleaved mass transfer layer form a subunit.

4. The device according to claim 3 having plural subunits.

5. The device according to claim 1 wherein the first lamina and the second lamina have a front side and a back side, and wherein the transfer layer is positioned between the back side of the first lamina and the front side of the second lamina.

6. The device according to claim 1 wherein the mass transfer layer is a membrane or semi-permeable membrane.

7. The device according to claim 1 wherein each of the first and second flow fields has a predominant direction of flow, and wherein a predominant direction of flow of the blood is countercurrent to a predominant direction of flow of the dialysate, or the direction of flow of the blood is crosscurrent to a predominant direction of flow of the dialysate.

8. The device according to claim 1 where the support structures (138) define substantially the same geometric shape, or the support structures define plural different geometric shapes.

9. The device according to claim 1, wherein the mass transfer layer is a semi-permeable membrane interleaving adjacent laminae.

10. The device according to claim 1 where the at least one mass transfer layer comprises a semi-permeable membrane, the device further comprises a heat transfer layer, or the device is configured for both heat transfer and mass transfer.

11. The device according to claim 1 where features defining a flow field have a predominant direction of fluid flow, and wherein the plural support structures are wall segments angled relative to the predominant direction of fluid flow.

12. The device according to claim 1, where the size decreases and the density increases in a predominant direction of fluid flow.

13. The device according to claim 1 comprising plural laminae defining microchannels, where the microchannels of each lamina are substantially parallel or substantially orthogonal to the microchannels of at least one adjacent lamina.

14. The device according to claim 13 where a substantial portion of the microchannels extend from the front side to the back side to form through-cut microchannels, the through-cut microchannels fluidly connected to one or more inlet vias by first partial thickness microchannels and the through-cut microchannels fluidly connected to the one or more outlet vias by second partial thickness microchannels.

## Patentansprüche

1. Mikrofluidik-Vorrichtung zur Durchführung von Dialyse, die aufweist:
eine erste Lamina, die ein erstes Fließfeld mit einem ersten Satz von Stützstrukturen aufweist, um die Blut während des Betriebs fließt;
eine zweite Lamina, die ein zweites Fließfeld (136) mit einem zweiten Satz von Stützstrukturen (138) aufweist, um die Dialysat während des Betriebs fließt, wobei jede der Stützstrukturen (138) im ersten Fließfeld und/oder zweiten Fließfeld (136) eine Säule aufweist, die einen Fuß mit einer Form hat, die aus einem Kreis, einem Tropfen, einem Dreieck, einer Ellipse oder einem Polygon ausgewählt ist, und wobei die Stützstrukturen im ersten Fließfeld und/oder zweiten Fließfeld (136) einen Größengradient und/oder einen Dichtegradient von einem Ende des Fließfelds (136) zum entgegengesetzten Ende des Fließfelds (136) haben; und
mindestens eine zwischen dem ersten und zweiten Fließfeld (136) verschachtelte Stofftransportschicht, über die es zu Dialyse des Bluts im Betrieb kommt,
**dadurch gekennzeichnet, dass**
die Stützstrukturen (138) im ersten Fließfeld und/oder zweiten Fließfeld (136) einen Größengradient und einen Dichtegradient im Fließfeld (136) haben, die von einem Ende des Fließfelds (136) zum entgegengesetzten Ende des Fließfelds (136) allmählich in der Größe ab- und in der Dichte zunehmen.

2. Vorrichtung nach Anspruch 1, wobei die Vorrichtung keinen Sammlerbereich aufweist, die Vorrichtung ferner einen Sammlerbereich aufweist, der mit dem ersten und/oder zweiten Fließfeld fluidverbunden ist, oder die Vorrichtung Sammlerbereiche aufweist, die sowohl mit dem ersten als auch mit dem zweiten Fließfeld fluidverbunden sind.

3. Vorrichtung nach Anspruch 1, wobei die erste und zweite Lamina zusammen mit der verschachtelten Stofftransportschicht eine Teileinheit bilden.

4. Vorrichtung nach Anspruch 3, die mehrere Teileinheiten hat.

5. Vorrichtung nach Anspruch 1, wobei die erste Lamina und die zweite Lamina eine Vorderseite und eine Rückseite haben und wobei die Transportschicht zwischen der Rückseite der ersten Lamina und der Vorderseite der zweiten Lamina positioniert ist.

6. Vorrichtung nach Anspruch 1, wobei die Stofftransportschicht eine Membran oder eine semipermeable Membran ist.

7. Vorrichtung nach Anspruch 1, wobei das erste und zweite Fließfeld jeweils eine vorherrschende Fließrichtung haben und wobei eine vorherrschende Fließrichtung des Bluts im Gegenstrom zu einer vorherrschenden Fließrichtung des Dialysats verläuft oder die Fließrichtung des Bluts im Querstrom zu einer vorherrschenden Fließrichtung des Dialysats verläuft.

8. Vorrichtung nach Anspruch 1, wobei die Stützstrukturen (138) im Wesentlichen die gleiche geometrische Form definieren oder die Stützstrukturen mehrere unterschiedliche geometrische Formen definieren.

9. Vorrichtung nach Anspruch 1, wobei die Stofftransportschicht eine semipermeable Membran ist, die benachbarte Laminae verschachtelt.

10. Vorrichtung nach Anspruch 1, wobei die mindestens eine Stofftransportschicht eine semipermeable Membran aufweist, die Vorrichtung ferner eine Wärmetransportschicht aufweist oder die Vorrichtung sowohl zum Wärmetransport als auch zum Stofftransport konfiguriert ist.

11. Vorrichtung nach Anspruch 1, wobei ein Fließfeld definierende Merkmale eine vorherrschende Fluidfließrichtung haben und wobei die mehreren Stützstrukturen Wandsegmente sind, die relativ zur vorherrschenden Fluidfließrichtung abgewinkelt sind.

12. Vorrichtung nach Anspruch 1, wobei in einer vorherrschenden Fluidfließrichtung die Größe ab- und die Dichte zunimmt.

13. Vorrichtung nach Anspruch 1 mit mehreren Laminae, die Mikrokanäle definieren, wobei die Mikrokanäle jeder Lamina im Wesentlichen parallel oder im Wesentlichen orthogonal zu den Mikrokanälen mindestens einer benachbarten Lamina sind.

14. Vorrichtung nach Anspruch 13, wobei sich ein wesentlicher Abschnitt der Mikrokanäle von der Vorderseite zur Rückseite erstreckt, um Durchstichmikrokanäle zu bilden, die Durchstichmikrokanäle mit einem oder mehreren Einlassdurchgängen durch erste Teildickenmikrokanäle fluidverbunden sind und die Durchstichmikrokanäle mit dem einen oder den mehreren Auslassdurchgängen durch zweite Teildickenmikrokanäle fluidverbunden sind.

## Revendications

1. Dispositif micro-fluidique pour réaliser une dialyse comprenant :
une première lame comprenant un premier champ d'écoulement comprenant un premier ensemble de structures de support autour desquelles le sang s'écoule, pendant le fonctionnement ;
une seconde lame comprenant un second champ d'écoulement (136) comprenant un second ensemble de structures de support (138) autour desquelles le dialysat s'écoule pendant le fonctionnement, dans lequel chacune des structures de support (138), dans au moins l'un parmi le premier champ d'écoulement et le second champ d'écoulement (136), comprend une colonne ayant une base avec une forme choisie parmi un cercle, une larme, un triangle, une ellipse ou un polygone, et dans lequel les structures de support, dans au moins l'un parmi le premier champ d'écoulement et le second champ d'écoulement (136), ont un gradient de taille et/ou un gradient de densité à partir d'une extrémité du champ d'écoulement (136) jusqu'à l'extrémité opposée du champ d'écoulement (136) ; et
au moins une couche de transfert de masse imbriquée entre les premier et second champs d'écoulement (136) couche au travers de laquelle, la dialyse du sang a lieu lorsqu'elle est en fonctionnement,
**caractérisé en ce que**
les structures de support (138) dans au moins l'un parmi le premier champ d'écoulement et le second champ d'écoulement (136) ont un gradient de taille et un gradient de densité dans le champ d'écoulement (136), diminuant progressivement en taille et augmentant progressivement en densité d'une extrémité du champ d'écoulement (136) à l'extrémité opposée du champ d'écoulement (136).

2. Dispositif selon la revendication 1, dans lequel le dispositif ne comprend pas de région collectrice, le dispositif comprend en outre une région collectrice raccordée de manière fluide à au moins l'un parmi le premier ou le second champ d'écoulement, ou le dispositif comprend en outre des régions collecterices raccordées de manière fluide à la fois au premier et au second champ d'écoulement.

3. Dispositif selon la revendication 1, dans lequel les première et seconde lames conjointement avec le couche de transfert de masse imbriquée forment une sous-unité.

4. Dispositif selon la revendication 3, ayant plusieurs sous-unités.

5. Dispositif selon la revendication 1, dans lequel la première lame et la seconde lame ont un côté avant et un côté arrière, et dans lequel la couche de transfert est positionnée entre le côté arrière de la première lame et le côté avant de la seconde lame.

6. Dispositif selon la revendication 1, dans lequel la couche de transfert de masse est une membrane ou une membrane semi-perméable.

7. Dispositif selon la revendication 1, dans lequel chacun des premier et second champs d'écoulement a une direction d'écoulement prédominante, et dans lequel la direction d'écoulement prédominante du sang est à contre-courant d'une direction d'écoulement prédominante du dialysat, ou la direction d'écoulement du sang est à courant transversal par rapport à la direction d'écoulement prédominante du dialysat.

8. Dispositif selon la revendication 1, dans lequel les structures de support (138) définissent sensiblement la même forme géométrique, ou les structures de support définissent plusieurs formes géométriques différentes.

9. Dispositif selon la revendication 1, dans lequel la couche de transfert de masse est une membrane semi-perméable s'imbriquant avec les lames adjacentes.

10. Dispositif selon la revendication 1, dans lequel la au moins une couche de transfert de masse comprend une membrane semi-perméable, le dispositif comprend en outre une couche de transfert de chaleur, ou le dispositif est configuré à la fois pour le transfert de chaleur et le transfert de masse.

11. Dispositif selon la revendication 1, dans lequel des caractéristiques définissant un champ d'écoulement ont une direction d'écoulement de fluide prédominante, et dans lequel la pluralité de structures de support sont des segments de paroi coudés par rapport à la direction prédominante d'écoulement du fluide.

12. Dispositif selon la revendication 1, dans lequel la taille diminue et la densité augmente dans une direction prédominante d'écoulement du fluide.

13. Dispositif selon la revendication 1, comprenant plusieurs lames définissant des micro-canaux, où les micro-canaux de chaque lame sont sensiblement parallèles ou sensiblement orthogonaux aux micro-canaux d'au moins une lame adjacente.

14. Dispositif selon la revendication 13, dans lequel une partie importante des micro-canaux s'étend à partir d'un côté avant jusqu'à un côté arrière pour former des micro-canaux découpés, les micro-canaux découpés étant raccordés de manière fluide à un ou plusieurs orifices d'entrée par des premiers micro-canaux d'épaisseur partielle et les micro-canaux découpés raccordés de manière fluide à un ou plusieurs orifices de sortie par des seconds micro-canaux d'épaisseur partielle.
